# EUROPEAN PATENT SPECIFICATION

(11) **EP 2 443 115 B1**
(45) Date of publication and mention of the grant of the patent: **28.08.2013**
(21) Application number: 10788597.2
(22) Date of filing: 17.06.2010
(51) Int. Cl.: C07D 417/14, C07D 417/10, C07D 417/02, C07D 413/14, C07D 413/10, C07D 413/02, A61K 31/437, A61K 31/4178, A61K 31/4155, A61P 37/06, A61P 37/04, A61P 37/00

(54) **Pyrrolo-pyridine derivatives useful to treat diseases connected to S1P1 receptors**
Pyrrolo-pyridine-derivate zur Behandlung von Krankheiten die mit den S1P1 Rezeptoren verbunden sind
Dérivés de pyrrolo-pyridine pour le traitement de maladies associées aux S1P1 réceptuers

(30) Priority: 19.06.2009 GB 0910689
(43) Date of publication of application: 25.04.2012
(73) Proprietor: Glaxo Group Limited, Brentford, Middlesex TW8 9GS (GB)
(72) Inventor: LIN, Xichen, Shanghai 201203 (CN); MENG, Qinghua, Shanghai 201203 (CN); ZHANG, Haibo, Shanghai 201203 (CN); LI, Chengyong, Shanghai 201203 (CN)
(74) Representative: Wilkinson, Johanna Elise
(86) International application number: PCT/CN2010/000867
(87) International publication number: WO 2010/145201

(56) References cited:
- EP-A1- 2 003 132
- WO-A1-2008/074821
- WO-A1-2008/076356
- WO-A1-2008/128951
- CN-A- 101 415 702

## Description

The present invention relates to novel compounds having pharmacological activity, processes for their preparation, pharmaceutical compositions containing them and compounds for use in the treatment of various disorders.

Sphingosine 1-phosphate (S1P) is a bioactive lipid mediator formed by the phosphorylation of sphingosine by sphingosine kinases and is found in high levels in the blood. It is produced and secreted by a number of cell types, including those of hematopoietic origin such as platelets and mast cells (Okamoto et al 1998 J Biol Chem 273(42):27104; Sanchez and Hla 2004, J Cell Biochem 92:913). It has a wide range of biological actions, including regulation of cell proliferation, differentiation, motility, vascularisation, and activation of inflammatory cells and platelets (Pyne and Pyne 2000, Biochem J. 349: 385). Five subtypes of S1P responsive receptor have been described, S1P1 (Edg-1), S1P2 (Edg-5), S1P3 (Edg-3), S1P4 (Edg-6), and S1P5 (Edg-8), forming part of the G-protein coupled endothelial differentiation gene family of receptors (Chun et al 2002 Pharmacological Reviews 54:265, Sanchez and Hla 2004 J Cellular Biochemistry, 92:913). These 5 receptors show differential mRNA expression, with S1P1-3 being widely expressed, S1P4 expressed on lymphoid and hematopoietic tissues and S1 P5 primarily in brain and to a lower degree in spleen. They signal via different subsets of G proteins to promote a variety of biological responses (Kluk and Hla 2002 Biochem et Biophysica Acta 1582:72, Sanchez and Hla 2004, J Cellular Biochem 92:913).

Proposed roles for the S1P1 receptor include lymphocyte trafficking, cytokine induction/suppression and effects on endothelial cells (Rosen and Goetzl 2005 Nat Rev Immunol. 5:560). Agonists of the S1 P1 receptor have been used in a number of autoimmune and transplantation animal models, including Experimental Autoimmune Encephalomelitis (EAE) models of MS, to reduce the severity of the induced disease (Brinkman et al 2003 JBC 277:21453; Fujino et al 2003 J Pharmacol Exp Ther 305:70; Webb et al 2004 J Neuroimmunol 153:108; Rausch et al 2004 J Magn Reson Imaging 20:16). This activity is reported to be mediated by the effect of S1P1 agonists on lymphocyte circulation through the lymph system. Treatment with S1P1 agonists results in the sequestration of lymphocytes within secondary lymphoid organs such as the lymph nodes, inducing a reversible peripheral lymphopoenia in animal models (Chiba et al 1998, J Immunology 160:5037, Forrest et al 2004 J Pharmacol Exp Ther 309:758; Sanna et al 2004 JBC 279:13839). Published data on agonists suggests that compound treatment induces loss of the S1 P1 receptor from the cell surface via internalisation (Graler and Goetzl 2004 FASEB J 18:551; Matloubian et al 2004 Nature 427:355; Jo et al 2005 Chem Biol 12:703) and it is this reduction of S1P1 receptor on immune cells which contributes to the reduction of movement of T cells from the lymph nodes back into the blood stream.

S1 P1 gene deletion causes embryonic lethality. Experiments to examine the role of the S1P1 receptor in lymphocyte migration and trafficking have included the adoptive transfer of labelled S1 P1 deficient T cells into irradiated wild type mice. These cells showed a reduced egress from secondary lymphoid organs (Matloubian et al 2004 Nature 427:355).

S1 P1 has also been ascribed a role in endothelial cell junction modulation (Allende et al 2003 102:3665, Blood Singelton et al 2005 FASEB J 19:1646). With respect to this endothelial action, S1 P1 agonists have been reported to have an effect on isolated lymph nodes which may be contributing to a role in modulating immune disorders. S1 P1 agonists caused a closing of the endothelial stromal 'gates' of lymphatic sinuses which drain the lymph nodes and prevent lymphocyte egress (Wei wt al 2005, Nat. Immunology 6:1228).

The immunosuppressive compound FTY720 (JP11080026-A) has been shown to reduce circulating lymphocytes in animals and man, have disease modulating activity in animal models of immune disorders and reduce remission rates in relapsing remitting Multiple Sclerosis (Brinkman et al 2002 JBC 277:21453, Mandala et al 2002 Science 296:346, Fujino et al 2003 J Pharmacology and Experimental Therapeutics 305:45658, Brinkman et al 2004 American J Transplantation 4:1019, Webb et al 2004 J Neuroimmunology 153:108, Morris et al 2005 EurJ Immunol 35:3570, Chiba 2005 Pharmacology and Therapeutics 108:308, Kahan et al 2003, Transplantation 76:1079, Kappos et at 2006 New Eng J Medicine 335:1124). This compound is a prodrug that is phosphorylated in vivo by sphingosine kinases to give a molecule that has agonist activity at the S1P1, S1P3, S1P4 and S1P5 receptors. Clinical studies have demonstrated that treatment with FTY720 results in bradycardia in the first 24 hours of treatment (Kappos et al 2006, New Eng J Medicine 335:1124). The bradycardia is thought to be due to agonism at the S1 P3 receptor, based on a number of cell based and animal experiments. These include the use of S1 P3 knockout animals which, unlike wild type mice, do not demonstrate bradycardia following FTY720 administration and the use of S1P1 selective compounds. (Hale et al 2004 Bioorganic & Medicinal Chemistry Letters 14:3501, Sanna et al 2004 JBC 279:13839, Koyrakh et al 2005 American J Transplantation 5:529)

Hence, there is a need for S1 P1 receptor agonist compounds with selectivity over S 1 P3 which might be expected to show a reduced tendency to induce bradycardia.

The following patent applications describe oxadiazole derivatives as S1 P1 agonists: WO03/105771, W005/058848, W006/047195, W006/100633, WO06/115188, WO06/131336, WO07/02492 and WO07/116866.

The following patent application describes indole-oxadiazole derivatives as antipicornaviral agents: WO96/009822. The following patent applications describe indole-carboxylic acid derivatives as leukotriene receptor antagonists, pesticides and agrochemical fungicides respectively: WO06/090817, EP 0 439 785 and DE 39 39 238.

International patent applications WO08/074821 and WO08/76356 describe oxadiazole-indole derivatives as S1P1 agonists.

A structurally novel class of compounds has now been found which provides agonists of the S1P1 receptor.

The present invention therefore provides compounds of formula (I) or a salt thereof: 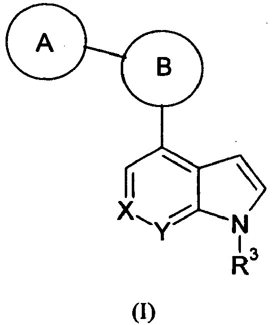 wherein
A is an aromatic ring selected from: 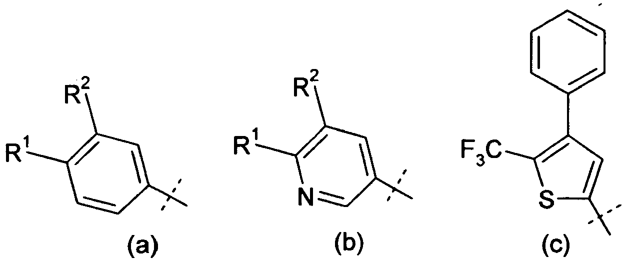 R¹ is C₍₁₋₆₎alkoxy or C(₁₋₆)alkyl;
R² is halogen, cyano or CF₃;
R³ is Z-COOH or C₍₁₋₆₎alkylOH;
B is a 5-membered heteroaryl ring selected from: 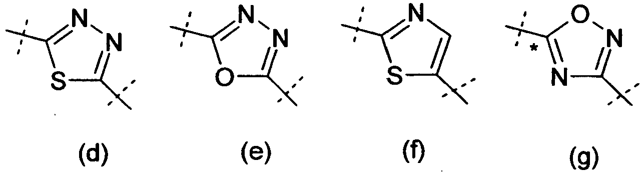 * indicates the point of attachment to the A ring;
Z is C₍₁₋₆₎alkyl or C₍₂₋₆₎alkenyl;
when Z is C₍₁₋₆₎alkyl it is optionally interrupted by a cyclopropyl, piperidinyl, azetidinyl, pyrrolidinyl, optionally interrupted by N or O and optionally substituted by O, cyclopropyl, halogen or methyl, when Z is C₍₂₋₆₎alkenyl it is optionally substituted by methyl; and
one of X and Y is CH and the other is N.

In one embodiment of the invention R¹ is C₍₁₋₃₎alkoxy. In another embodiment R¹ is isopropoxy.

In one embodiment of the invention R² is chloro or cyano. In another embodiment R² is chloro.

In one embodiment R³ is Z-COOH.

In one embodiment Z is C₍₁₋₆₎alkyl and is optionally interrupted by a cyclopropyl, piperidinyl, azetidinyl or pyrrolidinyl. In another embodiment Z is C₍₁₋₆₎alkyl optionally interrupted by N or O. In another embodiment Z is C₍₁₋₆₎alkyl substituted by O, cyclopropyl, halogen or methyl.

When Z is C₍₁₋₆₎alkyl interrupted by N or O it may also be substituted by methyl or O or methyl and O.

In another embodiment Z is C₍₂₋₃₎alkyl.

In one embodiment of the invention A is (a) or (b).

In one embodiment B is (d), (f) or (g). In another embodiment B is (g).

In one embodiment R³ is Z-COOH.

In one embodiment X is N and Y is CH. In another embodiment Y is N and X is CH.

In one embodiment of the invention
R¹ is C₍₁₋₃₎alkoxy;
R² is cyano or chloro;
R³ is Z-COOH; and
Z is C₍₂₋₃₎alkyl.
A is (a) or (b);
B is (d), (f) or (g): and
one of X and Y is CH and the other is N.

In one embodiment of the invention
R¹ is isopropoxy;
R² is cyano or chloro;
R³ is Z-COOH; and
Z is C₍₂₋₃₎alkyl.
A is (a) or (b);
B is (d), (f) or (g): and
One of X and Y is CH and the other is N.

The term "alkyl" as a group or part of a group e.g. alkoxy or hydroxyalkyl refers to a straight or branched alkyl group in all isomeric forms. The term "C₍₁₋₆₎ alkyl" refers to an alkyl group, as defined above, containing at least 1, and at most 6 carbon atoms Examples of such alkyl groups include methyl, ethyl, propyl, *iso*-propyl, *n*-butyl, *iso*butyl, *sec*-butyl, or *tert*-butyl. Examples of such alkoxy groups include methoxy, ethoxy, propoxy, *iso*-propoxy, butoxy, *iso*-butoxy, *sec*-butoxy and *tert*-butoxy.

The term "alkenyl" as a group or part of a group refers to a straight or branched alkenyl group in all isomeric forms. The term "C₍₂₋₆₎ alkenyl" refers to an alkenyl group, as defined above, containing at least 2, and at most 6 carbon atoms containing at least one carbon-to-carbon double bond. Examples of such alkenyl groups include ethenyl, propenyl and butenyl, pentenyl and hexenyl.

As used herein, the term "halogen" refers to fluorine (F), chlorine (Cl), bromine (Br), or iodine (I) and the term "halo" refers to the halogen: fluoro (-F), chloro (-Cl), bromo(-Br) and iodo(-I).

The term "heteroaryl" represents an unsaturated ring which comprises one or more heteroatoms selected from O, N or S. Examples of 5 or 6 membered heteroaryl rings include pyrrolyl, triazolyl, thiadiazolyl, tetrazolyl, imidazolyl, pyrazolyl, isothiazolyl, thiazolyl, isoxazolyl, oxazolyl, oxadiazolyl, furazanyl, furanyl, thienyl, pyridyl, pyrimidinyl, pyrazinyl, pyridazinyl and triazinyl.

In certain of the compounds of formula (I), dependent upon the nature of the substituent there are chiral carbon atoms and therefore compounds of formula (I) may exist as stereoisomers. The invention extends to all optical isomers such as stereoisomeric forms of the compounds of formula (I) including enantiomers, diastereoisomers and mixtures thereof, such as racemates. The different stereoisomeric forms may be separated or resolved one from the other by conventional methods of any given isomer may be obtained by conventional stereoselective or asymmetric syntheses.

Certain of the compounds herein can exist in various tautomeric forms and it is to be understood that the invention encompasses all such tautomeric forms.

Suitable compounds of formula (I) are:
3-[4-(5-{3-chloro-4-[(1-methylethyl)oxy]phenyl}-1,2,4-oxadiazo)-3-yl)-1*H*-pyrrolo[2,3-*b*]pyridin-1-yl]propanoic acid
3-[4-(5-{5-chloro-6-[(1-methylethyl)oxy]-3-pyridinyl}-1,2,4-oxadiazol-3-yl)-1H-pyrrolo[2,3-b]pyridin-1-yl]propanoic acid
4-[4-(5-{3-chloro-4-[(1-methylethyl)oxy]phenyl}-1,2,4-oxadiazol-3-yl)-1H-pyrrolo[2,3-b]pyridin-1-yl]butanoic acid
4-[4-(5-{5-chloro-6-[(1-methylethyl)oxy]-3-pyridinyl}-1,2,4-oxadiazol-3-yl)-1*H-*pyrrolo[2,3-b]pyridin-1-yl]butanoic acid
3-(4-(5-{3-chloro-4-[(1-methylethyl)oxy]phenyl}-1,2,4-oxadiazol-3-yl)-1*H*-pyrrolo[2,3-*c*]pyridin-1-yl]propanoic acid
4-[4-(5-{3-chloro-4-[(11-methylethyl)oxy]phenyl}-1,2,4-oxadiazol-3-yl)-1*H*-pyrrolo[2,3-*c*]pyridin-1-yl]butanoic acid
3-[4-(5-{5-chloro-6-[(1-methylethyl)oxy]-3-pyridinyl}-1,2,4-oxadiazol-3-yl)-1*H-*pyrrolo[2,3-c]pyridin-1-yl]propanoic acid
4-[4-(5-{5-chloro-6-[(1-methylethyl)oxy]-3-pyridinyl}-1,2,4-oxadiazol-3-yl)-1*H-*pyrrolo[2,3-c]pyridin-1-yl]butanoic acid
3-[4-(5-{3-Chloro-4-[(1-methylethyl)oxy]phenyl}-1,3,4-thiadiazol-2-yl)-1*H*-pyrrolo[2,3-b]pyridin-1-yl]propanoic acid
4-[4-(5-{3-Chloro-4-[(1-methy)ethyl)oxy]phenyl}-1,3,4-thiadiazo)-2-yl)-1*H*-pyrrolo[2,3-b]pyridin-1-yl]butanoic acid
3-[4-(5-{3-Chloro-4-[(1-methylethyl)oxy]phenyl}-1,3,4-oxadiazol-2-yl)-1*H*-pyrrolo[2,3-b]pyridin-1-yl]propanoic acid
4-[4-(5-{3-Chloro-4-[(1-methylethyl)oxy]phenyl}-1,3,4-oxadiazol-2-yl)-1*H*-pyrrolo[2,3-b]pyridin-1-yl]butanoic acid
3-[4-(2-{3-Chloro-4-[(1-methylethyl)oxy]phenyl}-1,3-thiazol-5-yl)-1*H*-pyrrolo[2,3-b]pyridin-1-yl]propanoic acid
4-[4-(2-{3-chloro-4-[(1-methylethyl)oxy]phenyl}-1,3-thiazol-5-yl)-1*H*-pyrrolo(2,3-b]pyridin-1-yl]butanoic acid
or salts thereof.

Pharmaceutically acceptable derivatives of compounds of formula (I) include any pharmaceutically acceptable salt, ester or salt of such ester of a compound of formula (I) which, upon administration to the recipient is capable of providing (directly or indirectly) a compound of formula (I) or an active metabolic or residue thereof.

The compounds of formula (I) can form salts. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. Certain of the compounds of formula (I) may form acid addition salts with one or more equivalents of the acid. The present invention includes within its scope all possible stoichiometric and non-stoichiometric forms. Salts may also be prepared from pharmaceutically acceptable bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic bases include salts of primary, secondary, and tertiary amines; substituted amines including naturally occurring substituted amines; and cyclic amines. Particular pharmaceutically acceptable organic bases include arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, procaine, purines, theobromine, triethylamine, trimethylamine, tripropylamine, tris(hydroxymethyl)aminomethane (TRIS, trometamol) and the like. Salts may also be formed from basic ion exchange resins, for example polyamine resins. When the compound of the present invention is basic, salts may be prepared from pharmaceutically acceptable acids, including inorganic and organic acids. Such acids include acetic, benzenesulfonic, benzoic, camphorsulfonic, citric, ethanesulfonic, ethanedisulfonic, fumaric, gluconic, glutamic, hydrobromic, hydrochloric, isethionic, lactic, maleic, malic, mandelic, methanesulfonic, mucic, pamoic, pantothenic, phosphoric, propionic, succinic, sulfuric, tartaric, p-toluenesulfonic acid, and the like.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

Included within the scope of the invention are all salts, solvates, hydrates, complexes, polymorphs, radiolabelled derivatives, stereoisomers and optical isomers of the compounds of formula (I).

In a further aspect, this invention provided processes for the preparation of a compound of formula (I). In one aspect, certain compounds of formula (I) were prepared by the process in Schemes 1 to 3.

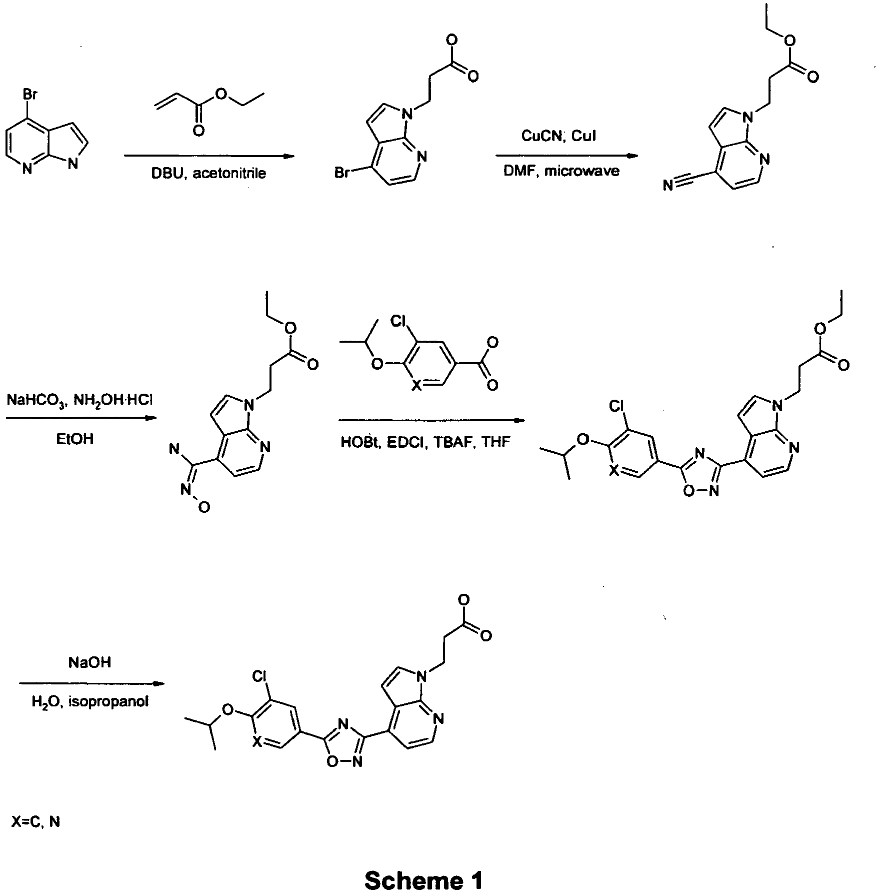

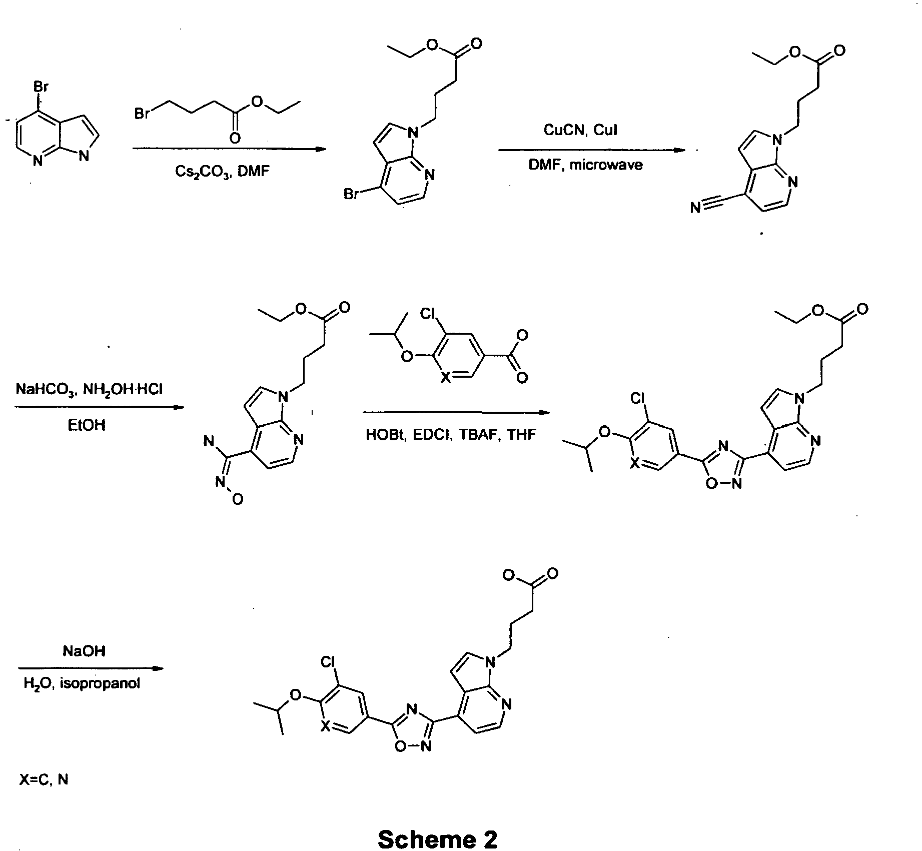

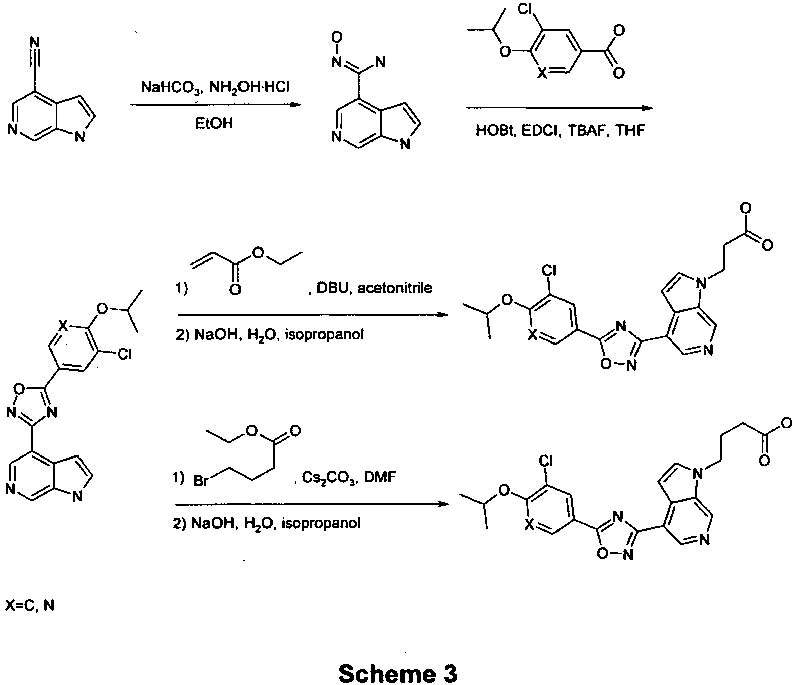

Pharmaceutically acceptable salts may be prepared conventionally by reaction with the appropriate acid or acid derivative.

The potencies and efficacies of the compounds of this invention for the S1 P1 receptor can be determined by GTPγS assay or S1P1 Tango assay performed on the human cloned receptor as described herein. Compounds of formula (I) have demonstrated agonist activity at the S1P1 receptor, using functional assays described herein.

Compounds of formula (I) and their pharmaceutically acceptable salts are therefore of use in the treatment of conditions or disorders which are mediated via the S1P1 receptor. In particular the compounds of formula (I) and their pharmaceutically acceptable salts are of use in the treatment of multiple sclerosis, autoimmune diseases, chronic inflammatory disorders, asthma, inflammatory neuropathies, arthritis, transplantation, Crohn's disease, ulcerative colitis, lupus erythematosis, psoriasis, ischemia-reperfusion injury, solid tumours, and tumour metastasis, diseases associated with angiogenesis, vascular diseases, pain conditions, acute viral diseases, inflammatory bowel conditions, insulin and non-insulin dependant diabetes.

Compounds of formula (I) and their pharmaceutically acceptable salts are therefore of use in the treatment of multiple sclerosis.

Compounds of formula (I) and their pharmaceutically acceptable salts may also be of use in the treatment of Parkinson's Disease, Alzheimer's disease, Huntington's chorea, amyotrophic lateral sclerosis, spinal muscular atrophy, polyglutamine expansion disorders, vascular dementia, Down's syndrome, HIV dementia, dementia, ocular diseases including glaucoma, aged related macular degeneration, cataracts, traumatic eye injury, diabetic retinopathy, traumatic brain injury, stroke, tauopathies and hearing loss.

It is to be understood that "treatment" as used herein includes prophylaxis as well as alleviation of established symptoms.

Thus the invention also provides a compound of formula (I) or a pharmaceutically acceptable salt thereof, for use as a therapeutic substance, in particular in the treatment of the conditions or disorders mediated via the S1P1 receptor. In particular the invention provides a compound of formula (I) or a pharmaceutically acceptable salt thereof for use as a therapeutic substance in the treatment of multiple sclerosis, autoimmune diseases, chronic inflammatory disorders, asthma, inflammatory neuropathies, arthritis, transplantation, Crohn's disease, ulcerative colitis, lupus erythematosis, psoriasis, ischemia-reperfusion injury, solid tumours, and tumour metastasis, diseases associated with angiogenesis, vascular diseases, pain conditions, acute viral diseases, inflammatory bowel conditions, insulin and non-insulin dependant diabetes.

Compounds of formula (I) and their pharmaceutically acceptable salts are of use as therapeutic substances in the treatment of multiple sclerosis.

In another aspect, the invention provides for the use of a compound of formula (I) or a pharmaceutically acceptable salt thereof in the manufacture of a medicament for use in the treatment of the conditions or disorders mediated via the S1 P1 receptor

In order to use the compounds of formula (I) and pharmaceutically acceptable salts thereof in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose); fillers (e.g. lactose, microcrystalline cellulose or calcium hydrogen phosphate); tabletting lubricants (e.g. magnesium stearate, talc or silica); disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitably formulated to give controlled release of the active compound.

For parenteral administration, fluid unit dosage forms are prepared utilising a compound of the invention or pharmaceutically acceptable salts thereof and a sterile vehicle. Formulations for injection may be presented in unit dosage form e.g. in ampoules or in multi-dose, utilising a compound of the invention or pharmaceutically acceptable derivatives thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of formula (I) or pharmaceutically acceptable salts thereof may also be formulated in rectal compositions such as suppositories or retention enemas, e.g. containing conventional suppository bases such as cocoa butter or other glycerides.

The compounds of formula (I) or pharmaceutically acceptable salts thereof may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of formula (I) or pharmaceutically acceptable salts thereof, may be formulated as solutions for administration via a suitable metered or unitary dose device or alternatively as a powder mix with a suitable carrier for administration using a suitable delivery device. Thus compounds of formula (I) or pharmaceutically acceptable salts thereof may be formulated for oral, buccal, parenteral, topical (including ophthalmic and nasal), depot or rectal administration or in a form suitable for administration by inhalation or insufflation (either through the mouth or nose).

The compounds of formula (I) or pharmaceutically acceptable salts thereof may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The composition may contain from 0.1% to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, 1.0 to 500mg or 1.0 to 200 mg and such unit doses may be administered more than once a day, for example two or three times a day.

Compounds of formula, (I) or pharmaceutically acceptable salts thereof may be used in combination preparations. For example, the compounds of the invention may be used in combination with cyclosporin A, methotrexate, steriods, rapamycin, proinflammatory cytokine inhibitors, immunomodulators including biologicals or other therapeutically active compounds.

The subject invention also includes isotopically-labeled compounds, which are identical to those recited in formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as ³H, ¹¹C, ¹⁴C, ¹⁸F, ¹²³I and ¹²⁵I.

Compounds of the present invention and pharmaceutically acceptable saltss of said compounds that contain the aforementioned isotopes and/or other isotopes of other atoms are within the scope of the present invention. Isotopically-labeled compounds of the present invention, for example those into which radioactive isotopes such as ³H, ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. "C and ⁸F isotopes are particularly useful in PET (positron emission tomography), and ¹²⁵I isotopes are particularly useful in SPECT (single photon emission computerized tomography), all useful in brain imaging. Further, substitution with heavier isotopes such as deuterium, i.e., ²H, can afford certain therapeutic advantages resulting from greater metabolic stability, for example increased *in vivo* half-life or reduced dosage requirements and, hence, may be preferred in some circumstances. Isotopically labelled compounds of formula (I) and following of this invention can generally be prepared by carrying out the procedures disclosed in the Schemes and/or in the Examples below, by substituting a readily available isotopically labelled reagent for a non-isotopically labeled reagent.

The following Descriptions and Examples illustrate the preparation of compounds of the invention.

### Abbreviations

| | |
|---|---|
| g - | grams |
| mg - | milligrams |
| ml - | millilitres |
| min - | minute |
| ul - | microlitres |
| MeCN - | acetonitrile |
| MeOH - | methanol |
| EtOH - | ethanol |
| Et₂O - | diethyl ether |
| EtOAc - | ethyl acetate |
| DABCO - | 1,4-diazabiclo[2,2,2]octane |
| DCM - | dichloromethane |
| DIAD - | diisopropyl azodicarboxylate |
| DME - | 1,2-bis(methyloxy)ethane |
| DMF - | *N*,*N*-dimethylformamide |
| DMSO - | dimethylsulphoxide |
| EDAC - | *N*-(3-Dimethylaminopropyl)-*N*'-ethylcarbodiimide hydrochloride |
| EDC - | *N*-(3-Dimethylaminopropyl)-*N*'-ethylcarbodiimide hydrochloride |
| EDCl - | *N*-(3-Dimethylaminopropyl)-*N*'-ethylcarbodiimide hydrochloride |
| HOBT/HOBt - | Hydroxybenzotriazole |
| IPA - | isopropylalcohol |
| NCS - | *N*-chlorosuccinimide |
| PyBOP - | Benzotriazol-1-yl-oxytripyrrolidinophosphonium hexafluorophosphate |
| THF - | tetrahydrofuran |
| dba - | dibenzylidene acetone |
| RT - | room temperature |
| °C - | degrees Celsius |
| M - | Molar |
| H - | proton |
| s - | singlet |
| d - | doublet |
| t - | triplet |
| q - | quartet |
| MHz - | megahertz |
| MeOD - | deuterated methanol |
| LCMS - | Liquid Chromatography Mass Spectrometry |
| LC/MS - | Liquid Chromatography Mass Spectrometry |
| MS - | mass spectrometry |
| ES - | Electrospray |
| MH⁺ - | mass ion + H⁺ |
| MDAP - | mass directed automated preparative liquid chromatography. |
| sat. - | saturated |

### Chromatography

Unless stated otherwise, all chromatography was carried out using silica columns.

### General chemistry section

The intermediates for the preparation of the examples may not necessarily have been prepared from the specific batch of precursor described.

### Description for D1

### Ethyl 3-(4-bromo-1H-pyrrolo[2,3-b]pyridin-1-yl)propanoate (D1)

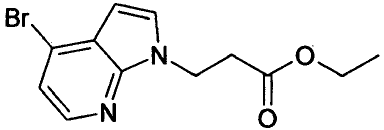

To a mixture of 4-bromo-1*H*-pyrrolo[2,3-b]pyridine (1.0 g) and 1,8-diazabicyclo [5.4.0]undec-7-ene (1.1 g) in acetonitrile (100 mL) at room temperature, was added ethyl 2-propenoate (3.5 g). The reaction mixture was heated to 70°C and stirred at that temperature for 12 hours. The mixture was concentrated to afford ethyl 3-(4-bromo-1*H*-pyrrolo[2,3-b]pyridin-1-yl)propanoate (D1) as light oil (3.0 g). MS (ES): C₁₂H₁₃BrN₂O₂ requires 296 (⁷⁹Br); 298 (⁸¹Br); found 297.0 (M+H⁺, ⁷⁹Br), 299.1 (M+H⁺, ⁸¹Br).

### Description for D2

### ethyl 3-(4-cyano-1H-pyrrolo[2,3-b]pyridin-1-yl)propanoate(D2)

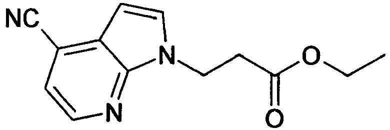

To a mixture of ethyl 3-(4-bromo-1*H*-pyrrolo[2,3-b]pyridin-1-yl)propanoate (D1) (1.0 g) and copper(I) cyanide (1.2 g) in dry DMF (15 mL) in a reaction vessel at room temperature, was added copper(I) iodide (513.0 mg). The reaction vessel was sealed and heated in Biotage Initiator using initial high to 180 °C for 100 min. The mixture was filtered and the filtered-cake was washed with DMF (10 mL) for 3 times. The filtration was evaporated and purified on column chromatography to afford the ethyl 3-(4-cyano-1H-pyrrolo[2,3-b]pyridin-1-yl)propanoate (D2) as a colourless oil (423.0 mg). δH (DMSO-*d₆*, 400MHz): 1.09 (3H, t), 2.93 (2H, t), 4.01 (2H, q), 4.58 (2H, t), 6.68 (1H, d), 7.61 (1 H, d), 7.92 (1H, d), 8.46 (1 H, d). MS (ES): C₁₃H₁₃N₃O₂ requires 243; found 244.1 (M+H⁺)

### Description for D3

### Ethyl 3-{4-[(Z)-(hydroxyamino)(imino)methy)]-1H-pyrrolo[2,3-b]pyridin-1-yl)propanoate(D3)

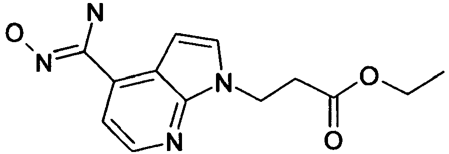

To a mixture of ethyl 3-(4-cyano-1H-pyrrolo[2,3-b]pyridin-1-yl)propanoate (D2) (423.0 mg) and hydroxylamine hydrochloride (242.0 mg) in ethanol (10 mL) at room temperature, was added sodium bicarbonate (440.0 mg). The reaction mixture was heated to 80°C and stirred at that temperature for 12 hours. The mixture was concentrated to afford 3-(4-[(Z)-(hydroxyamino)(imino)methyl]-1H-pyrrolo[2,3-b] pyridine-1-yl}propanoate (D3) as brown oil (480.0 mg). MS (ES): C₁₃H₁₆N₄O₃ requires 276; found 277.2 (M+H⁺)

### Description for D4

### ethyl 3-[4-(5-{3-chloro-4-[(1-methylethyl)oxy]phenyl}-1,2,4-oxadiazol-3-yl)-1H-pyrrolo[2,3-b]pyridin-1-yl]propanoate(D4)

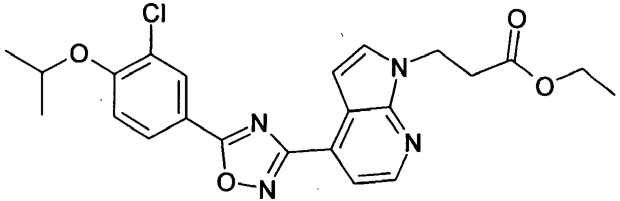

To a solution of 3-chloro-4-[(1-methylethyl)oxy]benzoic acid (D37) (187.0 mg) in THF (10 mL) at room temperature, was added HOBT (287.0 mg) and EDCI (330.0 mg). After the resulting solution was stirred for 30 min, 3-{4-[(Z)-(hydroxyamino)(imino) methyl]-1H-pyrrolo[2,3-b]pyridin-1-yl}propanoate (D3) (240.0 mg) was added to the reaction mixture and stirred at room temperature for 2 hour. TBAF (900.0 mg) was added the reaction mixture at room temperature. The reaction mixture was heated to 80 °C and stirred at that temperature for 16 hours. The mixture was concentrated and purified on column chromatography to afford 3-[4-(5-{3-chloro-4-[(1-methyl ethyl)oxy]phenyl}-1,2,4-oxadiazol-3-yl)-1H-pyrrolo[2,3-b]pyridin-1-yl]propanoate (D4) as brown oil (140.0 mg). MS (ES): C₂₃H₂₃ClN₄O₄ requires 454; found 455.2 (M+H⁺)

### Description for D5

### ethyl 3-[4-(5-{5-chloro-6-[(1-methylethyl)oxy]-3-pyridinyl}-1,2,4-oxadiazol-3-yl)-1H-pyrrolo[2,3-b]pyridin-1-yl]propanoate (D5)

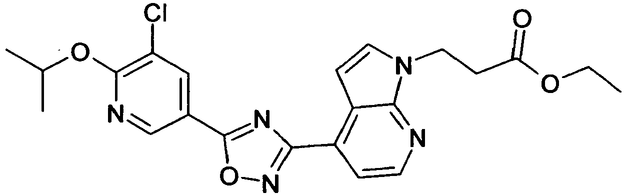

To a solution of 5-chloro-6-[(1-methylethyl)oxy]-3-pyridinecarboxylic acid (D38) (211.0 mg) in THF (20 mL) at room temperature, was added HOBt (323.0 mg) and EDCl (375.0 mg). After the reaction mixture was stirred at room temperature for 30 min, ethyl 3-{4-[(E)-(hydroxyamino)(imino)methyl]-1H-pyrrolo[2,3-b]pyridin-1-yl} propanoate (270.0 mg) (D3) was added the reaction mixture at room temperature and stirred at that temperature for 2h. TBAF (1.1 g) was added the reaction mixture at room temperature. The reaction mixture was heated to 90°C and stirred at that temperature for 60 hours. The mixture was concentrated and purified on column chromatography to afford ethyl 3-[4-(5-{5-chloro-6-[(1-methylethyl)oxy]-3-pyridinyl}-1,2,4-oxadiazol -3-yl)-1H-pyrrolo[2,3-b]pyridin-1-yl]propanoate (D5) as white solid (211.0 mg). MS (ES): C₂₂H₂₂ClN₅O₄ requires 455; found 456.2 (M+H⁺)

### Description for D6

### ethyl 4-(4-bromo-1H-pyrrolo[2,3-b]pyridin-1-yl)butanoate (D6)

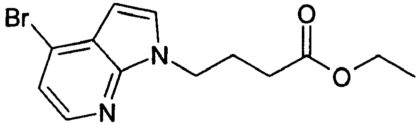

To a mixture of 4-bromo-1H-pyrrolo[2,3-b]pyridine (400.0 mg), Cs₂CO₃ (3.7 g) and KI (23.0 mg) in DMF (20 mL) at room temperature, was added ethyl 4-bromobutanoate (2.2 g). The reaction mixture was heated to 80°C and stirred at that temperature for 16 hours. The mixture was filtered and the filtered-cake was washed with DMF (2 mL) for 2 times. Then the filtration was concentrated to afford ethyl 4-(4-bromo-1H-pyrrolo[2,3-b]pyridin-1-yl)butanoate (D6) as brown oil (1.5 g). MS (ES): C₁₃H₁₅BrN₂O₂ requires 310, ⁷⁹Br; 312, ⁸¹Br; found 311.0, ⁷⁹Br; 313.1, ⁸¹Br (M+H⁺)

### Description for D7

### Ethyl 4-(4-cyano-1H-pyrrolo[2,3-b]pyridin-1-yl)butanoate (D7)

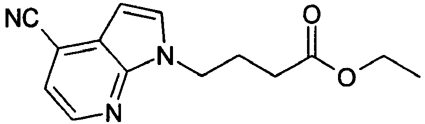

To a mixture of ethyl 4-(4-bromo-1H-pyrrolo[2,3-b]pyridin-1-yl)butanoate (D6) (870.0 mg) and copper(I) cyanide (1.0 g) in dry DMF (15 mL) in a reaction vessel at room temperature, was added copper(I) iodide (426.0 mg). The reaction vessel was sealed and heated in Biotage Initiator using initial high to 180 °C for 100 min. The mixture was filtered and the filtered-cake was washed with DMF (10 mL) for 3 times The filtration was evaporated and purified on column chromatography to afford the Ethyl 4-(4-cyano-1H-pyrrolo[2,3-b]pyridin-1-yl)butanoate (D7) as a brown oil (353.0 mg).
MS (ES): C₁₄H₁₅N₃O₂ requires 257; found 258.2 (M+H⁺)

### Description for D8

### ethyl 4-{4-[(Z)-(hydroxyamino)(imino)methyl]-1H-pyrrolo[2,3-b]pyridin-1-yl} butanoate (D8)

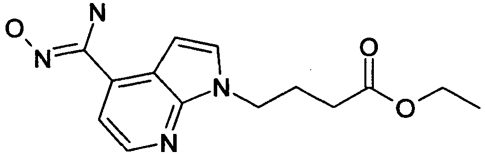

To a mixture of Ethyl 4-(4-cyano-1H-pyrrolo[2,3-b]pyridin-1-yl)butanoate (D7) (353.0 mg) and hydroxylamine hydrochloride (199.0 mg) in ethanol (10 mL) at room temperature, was added sodium bicarbonate (360.0 mg). The reaction mixture was heated to 80°C and stirred at that temperature for 12 hours. The mixture was concentrated to afford 4-{4-[(Z)-(hydroxyamino)(imino)methyl]-1H-pyrrolo[2,3-b] pyridin-1-yl)butanoate (D8) as brown oil (380.0 mg). MS (ES): C₁₄H₁₈N₄O₃ requires 290; found 291.2 (M+H⁺)

### Description for D9

### ethyl 4-[4-{5-{3-chloro-4-[(1-methylethyl)oxy]phenyl}-1,2,4-oxadiazol-3-yl)-1H-pyrrolo[2,3-b]pyridin-1-yl]butanoate (D9)

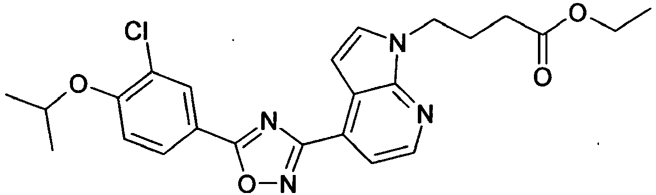

To a solution of 3-chloro-4-[(1-methylethyl)oxy]benzoic acid (D37) (137 mg) in THF (20 mL) at room temperature, were added HOBT (211.0 mg) and EDCl (245.0 mg). After the resulting solution was stirred for 30 min at room temperature, ethyl 4-{4-[(*Z*)-(hydroxyamino)(imino)methyl]-1*H*-pyrrolo[2,3-*b*]pyridin-1-yl}butanoate (D8) (185.0 mg) was added and the reaction mixture at room temperature and stirred at that temperature for 2 hour. TBAF (705.0 mg) was added the reaction mixture at room temperature. The reaction mixture was heated to 80 °C and stirred at that temperature for 60 h. The mixture was concentrated and the residue were dissolved in DMF and purified by Mass Directed AutoPrep. The solvent was evaporated in vacuo to afford ethyl 4-[4-(5-{3-chloro-4-[(1-methylethyl)oxy]phenyl}-1,2,4-oxadiazol-3-yl)-1H-pyrrolo [2,3-b]pyridin-1-yl]butanoate (D9) as white solid (173.0 mg). MS (ES): C₂₄H₂₅ClN₄O₄ requires 468; found 469.2 (M+H⁺)

### Description for D10

### Ethyl 4-[4-(5-{5-chloro-6-[(1-methylethyl)oxy]-3-pyridinyl}-1,2,4-oxadiazol-3-yl)-1H-pyrrolo[2,3-b]pyridin-1-yl]butanoate (D10)

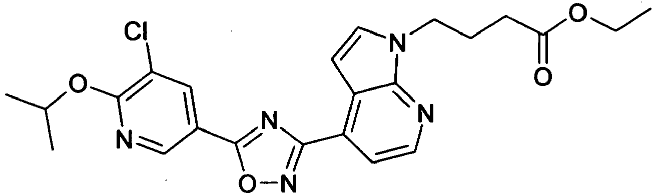

To a solution of 5-chloro-6-[(1-methylethyl)oxy]-3-pyridinecarboxylic acid (D38) (148.7 mg) in Tetrahydrofuran (THF) (20 mL) at room temperature, was added HOBt (227.6 mg) and EDCI (264.3 mg). After the reaction mixture was stirred at room temperature for 30 min, ethyl 4-{4-[(Z)-(hydroxyamino)(imino)methyl]-1*H*-pyrrolo[2,3-b]pyridine -1-yl}butanoate (D8) (200.0 mg) was added the reaction mixture at room temperature and stirred at that temperature for 2 hours. TBAF (761.4 mg) was added the reaction mixture at room temperature. The reaction mixture was heated to 90°C and stirred at that temperature for 48 hours. After the mixture was concentrated, the residue were dissolved in DMF and purified by Mass Directed AutoPrep. The solvent was evaporated in vacuo to afford ethyl 4-[4-(5-{5-chloro-6-[(1-methylethyl)oxy] -3-pyridinyl}-1,2,4-oxadiazol-3-yl)-1H-pyrrolo[2,3-b]pyridin-1-yl]butanoate (D10) as white solid (260.0 mg). MS (ES): C₂₃H₂₄ClN₅O₄ requires 469; found 470.2 (M+H⁺)

### Description for D11

### 3-bromo-5-nitro-4-pyridinol (D11)

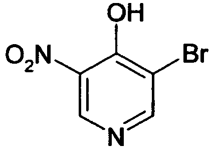

To a suspension of 3-nitro-4-pyridinol (40 g) in water (200 ml) was slowly added Br₂ (18.4 ml) dropwise at room temperature. Then the mixture was heated to 50°C and stirred at that temperature for 2 h. The mixture was cooled to room temperature. The resulting precipitate was filtered, washed with water, and dried to afford 3-bromo-5-nitro-4-pyridinol (D11) as a white solid (46 g). δH (DMSO-d₆, 400MHz): 8.81 (1H, s), 8.36 (1 H, s);

### Description for D12

### 3-bromo-4-chloro-5-nitropyridine (D12)

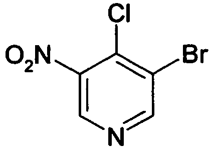

To a suspension of 3-bromo-5-nitro-4-pyridinol (D11) (25 g) in toluene (50 ml) was added POCl₃ (53 ml) at room temperature. The mixture was slowly heated to 100°C and stirred at that temperature overnight. The mixture was cooled to room temperature and concentrated. The resulting residue was carefully added ice-water, then extracted with EtOAc. The organic layers were separated, washed with water and brine, dried over anhydrous Na₂SO₄ and evaporated to afford 3-bromo-4-chloro-5-nitropyridine (D12) as a tan solid (23 g).

### Description for D13

### diethyl (3-bromo-5-nitro-4-pyridinyl)propanedioate (D13)

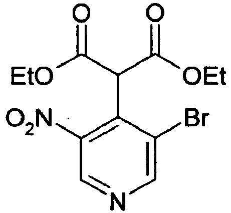

To a suspension of NaH (0.8 g, 60% in oil) in DMF (13 ml) was added diethyl malonate(3.2 g) dropwise at 0°C and stirred at that temperature for 0.5h, then 3-bromo-4-chloro-5-nitropyridine (D12) (2.4 g) was slowly added at 0 °C. The resulting solution was stirred at room temperature for 1 h, and then the mixture of diethyl (3-bromo-5-nitro-4-pyridinyl)propanedioate (D13) was used directly in the next step without further purification.

### Description for D14

### 3-bromo-4-methyl-5-nitropyridine (D14)

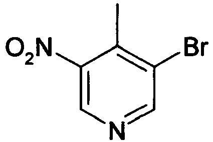

To the above mixture (D13) was added 9 N HCl (20 mL), then was heated to 100 °C and stirred at that temperature overnight. The reaction mixture was cooled to room temperature, and basified to pH=7 with 2 M NaOH aq.. The aqueous mixture was extracted with EtOAc. The organic layers were combined, washed with water and brine, dried over anhydrous Na₂SO₄ and evaporated to afford 3-bromo-4-methyl-5-nitropyridine (D14) as a yellow solid (1.3 g).

### Description for D15

### (E)-2-(3-bromo-5-nitro-4-pyridinyl)-N,N-dimethylethenamine (D15)

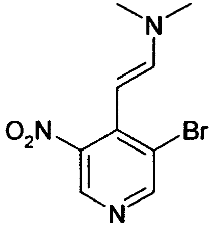

To a solution of 3-bromo-4-methyl-5-nitropyridine (D14) (3.8 g) in DMF (22 ml) was added N, N-dimethylformamide dimethyl acetal (4.7 ml), and the solution was heated to 90 °C for 1.5 h. The reaction mixture was cooled to room temperature and diluted with EtOAc. The organic layers were separated, washed with water and brine, dried over anhydrous Na₂SO₄ and evaporated to give (E)-2-(3-bromo-5-nitro-4-pyridinyl)-N,N-dimethylethenamine (D15) as a blood-red solid (4.6 g).

### Description for D16

### 4-bromo-1H-pyrrolo[2,3-c]pyridine (D16)

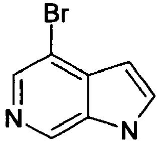

A mixture of compound (E)-2-(3-bromo-5-nitro-4-pyridinyl)-N,N-dimethylethenamine (D15) (1.2 g) and Zn (1.4 g) in acetic acid (25 ml) was refluxed and stirred for 45 mins, and cooled to room temperature. The mixture was diluted with EtOAc, filtered and the filtrate was basified to pH=8 with sat. aqueous NaHCO₃, and the aqueous layer was extracted with EtOAc, The organic layers were separated, washed with water and brine, dried over anhydrous Na₂SO₄ and evaporated to afford 4-bromo-1*H-*pyrrolo[2,3-*c*]pyridine (D16) as a brown oil (0.85 g). MS (ES): C₇H₄BrN₂ requires 195, ⁷⁹Br; 197, ⁸¹Br; found 196.8, ⁷⁹Br; 198.8, ⁸¹Br (M+H⁺)

### Description for D17

### 1H-pyrrolo[2,3-c]pyridine-4-carbonitrile (D17)

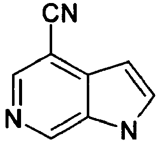

To a mixture of compound 4-bromo-1*H*-pyrrolo[2,3-c]pyridine (D16) (20.4 g), Zn(CN)₂ (12.2 g) and Zn dust (1.4 g) in DMF (300 mL) was added Pd(dppf)Cl₂ (8.5 g). The reaction mixture was heated to 120 °C and stirred at that temperature for 2 h. The mixture was diluted with water, extracted with EtOAc. The organic layers were washed with water and brine, dried over anhydrous Na₂SO₄, evaporated and purified on column chromatography to afford 1*H*-pyrrolo[2,3-c]pyridine-4-carbonitrile (D17) as white solid (3.9 g). δH (DMSO-*d₆*, 400MHz): 6.69 (1 H, d), 7.93 (1 H, d), 8.57 (1H, s), 9.01 (1 H, s), 12.30 (1H, s); MS (ES): C₈H₅N₃ requires 143; found 144.0 (M+H⁺)

### Description for D18

### N-hydroxy-1H-pyrrolo[2,3-c]pyridine-4-carboximidamide (D18)

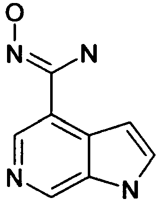

To a mixture of 1*H*-pyrrolo[2,3-*c*]pyridine-4-carbonitrile (D17) (400.0 mg) and hydroxylamine hydrochloride (388 mg) in ethanol (50 mL) at room temperature, was added sodium bicarbonate (704.0 mg). The reaction mixture was heated to 80°C and stirred at that temperature for 12 hours. The mixture was concentrated to afford N-hydroxy-1H-pyrrolo[2,3-c]pyridine-4-carboximidamide (D18) as white solid (518.0 mg). MS (ES): C₈H₈N₄O requires 176; found 177.1 (M+H⁺)

### Description for D19

### 4-(5-{3-chloro-4-[(1-methylethyl)oxy]phenyl}-1,2,4-oxadiazol-3-yl)-1H-pyrrolo[2,3-c̅]pyridine (D19)

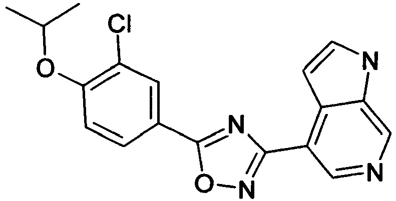

To a solution of 3-chtoro-4-[(1-methylethyl)oxy]benzoic acid (D37) (599.0 mg) in THF (40 mL) at room temperature, were added HOBT (0.9 g) and EDCl (1.1 g). After the resulting solution was stirred at room temperature for 30 min, N-hydroxy-1*H-*pyrrolo[2,3-c]pyridine-4-carboximidamide (D18) (492.0 mg) was added to the reaction mixture and stirred at that temperature for 2 hour. TBAF (3.1 g) was added to the reaction mixture at room temperature. The reaction mixture was heated to 90 °C and stirred at that temperature for 72 h. The mixture was concentrated and purified on column chromatography to afford 4-(5-{3-chloro-4-[(1-methylethyl)oxy] phenyl}-1,2,4-oxadiazol-3-yl)-1H-pyrrolo[2,3-c]pyridine (D19) as white solid (488.0 mg). MS (ES): C₁₈H₁₅ClN₄O₂ requires 354; found 355.1 (M+H⁺).

### Description for D20

### Ethyl 3-[4-(5-{3-chloro-4-[(1-methylethyl)oxy]phenyl}-1,2,4-oxadiazol-3-yl)-1H-pyrrolo[2,3-c]pyridin-1-yl]propanoate (D20)

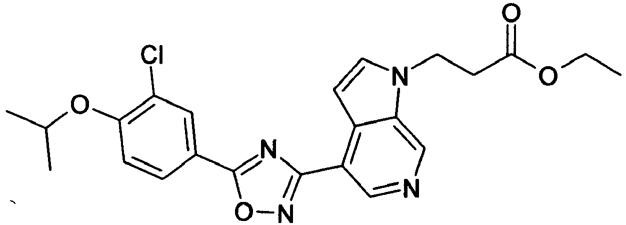

To a mixture of ethyl 2-propenoate (353 mg) and 4-(5-{3-chloro-4-[(1-methylethyl)oxy] phenyl}-1,2,4-oxadiazol-3-yl)-1H-pyrrolo[2,3-c]pyridine (D19) (250 mg) in acetonitrile (50 mL) at room temperature, was added 1,8-diazabicyclo[5.4.0]undec-7-ene (107.0 mg). The reaction mixture was heated to 82°C and stirred at that temperature for 12 hours. The mixture was concentrated and purified on column chromatography to afford ethyl 3-[4-(5-{3-chloro-4-[(1-methylethyl)oxy]phenyl}-1,2,4-oxadiazol-3-yl) -1H-pyrrolo[2,3-c]pyridin-1-yl]propanoate (D20) as white solid (280.0 mg). δH (DMSO-d₆, 400MHz): 1.08 (3H, t), 1.36 (6H, d), 2.95 (2H, t), 4.00 (2H, q), 4.64 (2H, t), 4.89 (1H, m), 7.03 (1 H, d), 7.46 (1 H, d), 7.83 (1 H, d), 8.15 (1 H, dd), 8.23 (1H, d), 8.93 (1 H, s), 9.12 (1 H, s). MS (ES): C₂₃H₂₃ClN₄O₄ requires 454; found 455.2 (M+H⁺).

### Description for D21

### Ethyl 4-[4-(5-{3-chloro-4-[(1-methylethyl)oxy]phenyl}-1,2,4-oxadiazol-3-yl)-1H-pyrrolo[2,3-c]pyridin-1-yl]butanoate (D21)

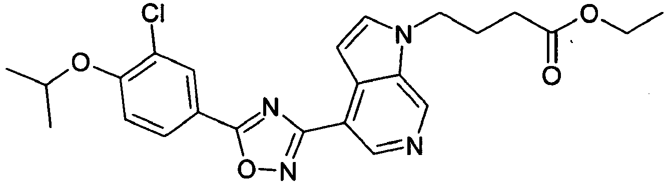

To a mixture of Kl (5.6 mg), Cs₂CO₃ (882.0 mg) and 4-(5-{3-chloro-4- [(1-methylethyl)oxy]phenyl}-1,2,4-oxadiazol-3-yl)-1H-pyrrolo[2,3-c]pyridine (D19) (240.0 mg) in N,N-Dimethylformamide (DMF) (30 mL) at room temperature, was added ethyl 4-bromobutanoate (528.0 mg). The reaction mixture was heated to 80°C and stirred at that temperature for 2 hours. The mixture was concentrated and purified on column chromatography to afford ethyl 4-[4-(5-{3-chloro-4-[(1-methylethyl)oxy] phenyl}-1,2,4-oxadiazol-3-yl)-1H-pyrrolo[2,3-c]pyridin-1-yl]butanoate (D21) as brown solid (177.0 mg). δH (DMSO-*d₆*, 400MHz): 1.12 (3H, t), 1.36 (6H, d), 2.09 (2H, m), 2.30 (2H, t), 3.99 (2H, q), 4.42 (2H, d), 4.88 (1H, m), 7.04(1H, d), 7.46 (1H, d), 7.84 (1H, d), 8.15 (1H, dd), 8.23 (1H, d), 8.93 (1H, s), 9.10 (1H, s). MS (ES): C₂₄H₂₅ClN₄O₄ requires 468; found 469.2 (M+H⁺).

### Description for D22

### 4-(5-{5-chloro-6-[(1-methylethyl)oxy]-3-pyridinyl}-1,2,4-oxadiazol-3-yl)-1H-pyrrolo[2,3-c]pyridine (D22)

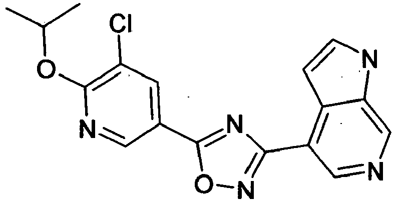

To a solution of 5-chloro-6-[(1-methylethyl)oxy]-3-pyridinecarboxylic acid (D38) (0.9 g) in THF (40 mL) at room temperature, were added HOBT (1.4 g) and EDCI (1.6 g). After the reaction mixture was stirred at room temperature for 30 min, *N*-hydroxy-1*H-*pyrrolo[2,3-c]pyridine-4-carboximidamide (D18) (0.7 g) was added at room temperature and stirred at room temperature for 2 hour. TBAF (4.6 g) was added to the reaction mixture at room temperature. The reaction mixture was heated to 90 °C and stirred at that temperature for 48 h. Then mixture was concentrated and purified on column chromatography to afford 4-(5-{5-chloro-6-[(1-methylethyl)oxy] -3-pyridinyl}-1,2,4-oxadiazol-3-yl)-1*H*-pyrrolo[2,3-c]pyridine (D22) as brown solid (0.6 g). MS (ES): C₁₇H₁₄ClN₅O₂ requires 355; found 356.1 (M+H⁺).

### Description for D23

### Ethyl 3-[4-(5-{5-chloro-6-[(1-methylethyl)oxy]-3-pyridinyl}-1,2,4-oxadiazol-3-yl)-1H-pyrrolo[2,3-c]pyridin-1-yl]propanoate (D23)

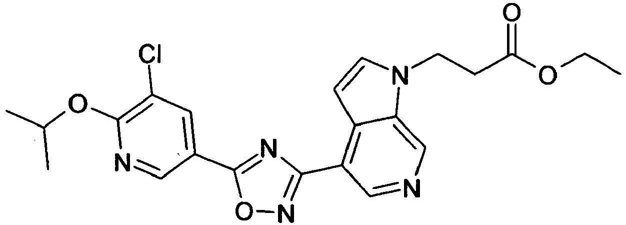

To a mixture of ethyl 2-propenoate (408.0 mg) and 4-(5-{5-chloro-6-[(1-methyl ethyl)oxy]-3-pyridinyl}-1,2,4-oxadiazol-3-yl)-1H-pyrrolo[2,3-c]pyridine (D22) (290.0 mg) in acetonitrile (50.0 mL) at room temperature, was added 1,8-diazabicyclo[5.4.0]undec-7-ene (124.0 mg). The reaction mixture was heated to 80°C and stirred at that temperature for 12 hours. The mixture was concentrated and purified on column chromatography to afford ethyl 3-[4-(5-{5-chloro-6- [(1-methylethyl)oxy]-3-pyridinyl}-1,2,4-oxadiazol-3-yl)-1H-pyrrolo[2,3-c]pyridin-1-yl] propanoate (D23) as white solid (331.0 mg). δH (DMSO-d₆, 400MHz): 1.08 (3H, t), 1.39 (6H, d), 2.95 (2H, t), 4.00 (2H, q), 4.64 (2H, t), 5.44 (1H, m), 7.03 (1H, d), 7.83(1H, d), 8.57 (1H, d), 8.92 (1H, s), 8.95 (1H, d), 9.12 (1H, s). MS (ES): C₂₂H₂₂ClN₅O₄ requires 455; found 456.2 (M+H⁺).

### Description for D24

### Ethyl 4-[4-(5-{5-chloro-6-[(1-methylethyl)oxy]-3-pyridinyl}-1,2,4-oxadiazol-3-yl)-1H-pyrrolo[2,3-c]pyridin-1-yl]butanoate (D24)

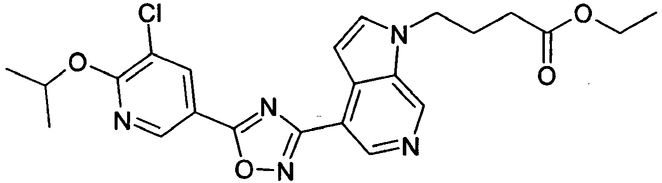

To a mixture of KI (7.5 mg), Cs₂CO₃ (1.2 g) and 4-(5-(5-chloro-6-[(1-methylethyl)oxy]-3-pyridinyl}-1,2,4-oxadiazol-3-yl)-1H-pyrrolo[2,3-c]pyridine (D22) (321.0 mg) and in N,N-Dimethylformamide (DMF) (30 mL) at room temperature, was added ethyl 4-bromobutanoate (528.0 mg). The reaction mixture was heated to 80°C and stirred at that temperature for 3 hours. The mixture was concentrated and purified on column chromatography to afford ethyl 4-[4-(5-{5-chloro-6-[(1-methylethyl)oxy]-3-pyridinyl}-1,2,4-oxadiazol-3-yl)-1H-pyrrolo[2,3-c]pyridin-1-yl]butanoate (D24) as brown solid (290.0 mg). δH (DMSO-d₆, 400MHz): 1.13 (3H, t), 1.39 (6H, d), 2.09 (2H, m), 2.30 (2H, t), 3.99 (2H, q), 4.42 (2H, t), 5.46 (1H, m), 7.06 (1 H, d), 7.85 (1H, d), 8.61 (1 H, d), 8.95 (1H, s), 8.98 (1H, d), 9.11 (1H, s). MS (ES): C₂₃H₂₄ClN₅O₄ requires 469; found 470.2 (M+H⁺).

### Description for D25

### 2-Bromo-5-{3-chloro-4-[(1-methylethyl)oxy]phenyl}-1,3,4-thiadiazole (D25)

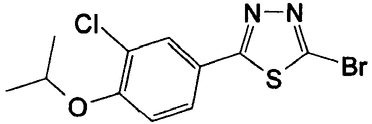

The mixture of 3-Chloro-4-[(1-methylethyl)oxy]benzoic acid (D37) (3.0 g), thiosemicarbazide (1.42 g) and phosphorus oxychloride (20 mL) were stirred at 90 °C under nitrogen for 3 hours. The reaction mixture was allowed to cool to room temperature and phosphorus oxychloride removed in vacuo. The resulting yellow oil was redissolved in EtOAc (while sat in an ice bath) and washed with water, dried on sodium sulfate, concentrated under vacuum to give crude {3-chloro-4-[(1-methylethyl)oxy]phenyl}-1,3,4-thiadiazol-2-amine. To a stirred mixture of crude 5-{3-chloro-4-[(1-methylethyl)oxy]phenyl}-1,3,4-thiadiazol-2-amine and copper(II) bromide (6.25 g) in acetonitrile (150 mL), 1,1-dimethylethyl nitrite (3.32 mL) was added at room temperature and the reaction mixture was stirred for 3 hours. The reaction mixture was diluted with EtOAc (200 mL) and 1M HCl (50 mL) was added. Organic phase was washed with water, saturated sodium bicarbonate, dried over anhydrous magnesium sulfate and the solvent removed under reduced pressure. The residue was purified by chromatography on silica gel to afford 2-bromo-5-{3-chloro-4-[(1-methylethyl)oxy]phenyl}-1,3,4-thiadiazole (D25) (2.6 g) as a yellow solid. δH (CDCl₃, 400 MHz): 1.41 (6H, d), 4.67 (1 H, m), 7.00 (1 H, d), 7.74 (1H, dd), 7.91 (1H, d). MS (ES): C₁₁H₁₀BrClN₂OS requires 332; found 333.0 (M+H⁺).

### Description for D26

### 2-Bromo-5-{3-chloro-4-[(1-methylethyl)oxy]phenyl}-1,3,4-oxadiazole (D26)

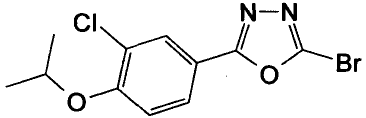

The mixture of 3-Chloro-4-[(1-methylethyl)oxy]benzoic acid (D37) (3.0 g), hydrazinecarboxamide hydrochloride (1.28 g) and phosphorus oxychloride (20 mL) were stirred at 90 °C under nitrogen for 3 hours. The reaction mixture was allowed to cool to room temperature and phosphorus oxychloride removed in vacuo. The resulting yellow oil was redissolved in EtOAc (while sat in an ice bath) and washed with water, dried on sodium sulfate, concentrated under vacuum to give crude 5-{3-chloro-4-[(1-methylethyl)oxy]phenyl}-1,3,4-oxadiazol-2-amine. To a stirred mixture of crude 5-{3-chloro-4-[(1-methylethyl)oxy]phenyl}-1,3,4-oxadiazol-2-amine and copper(II) bromide (6.25 g) in acetonitrile (150 mL), 1,1-dimethylethyl nitrite (3.32 mL) was added at room temperature and the reaction mixture was stirred for 3 hours. The reaction mixture was diluted with EtOAc (200 mL) and 1 M HCl (50 mL) was added. Organic phase was washed with water, saturated sodium bicarbonate, dried over anhydrous magnesium sulfate and the solvent removed under reduced pressure. The residue was purified by chromatography on silica gel to afford 2-bromo-5-{3-chloro-4-[(1-methylethyl)oxy]phenyl}-1,3,4-oxadiazole (D26) (2.5 g). δH (CDCl₃, 400 MHz): 1.41 (6H, d), 4.69 (1H, m), 7.02 (1H, d), 7.88 (1H, dd), 8.03 (1H, d). MS (ES): C₁₁H₁₀BrClN₂O₂ requires 316; found 317.0 (M+H⁺).

### Description for D27

### 2-{3-Chloro-4-[(1-methylethyl)oxy]phenyl}-1,3-thiazole (D27)

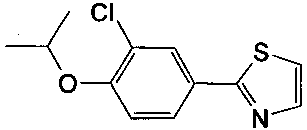

To a suspension of {3-chloro-4-[(1-methylethyl)oxy]phenyl}boronic acid (523 mg), 2-bromo-1,3-thiazole (400 mg) and Cs₂CO₃ (953 mg) in acetonitrile/water (4 mL/1 mL) was added PdCl₂(dppf)-CH₂Cl₂ adduct (199 mg). The mixture was purged with N₂, sealed and heated in microwave reactor to 120 °C for 0.5 h. After cooling the reaction, the mixture was diluted with methanol and filtered through Celite, the filtrate was concentrated and the residue was diluted with ethyl acetate and washed with water. The organic phase was dried over anhydrouos sodium sulfate. After removing the solvent, the crude product was purified by ISCO (10% ethyl acetate in hexanes) to afford 2-{3-chloro-4-[(1-methylethyl)oxy]phenyl}-1,3-thiazole (D27) (550 mg). δH (CDCl₃, 400MHz): 1.42 (6H, d), 4.64 (1 H, m), 7.00 (1H, d), 7.29 (1 H, d), 7.80 (1 H, d), 7.83 (1 H, dd), 8.00 (1 H, d). MS (ES): C₁₂H₁₂ClNOS requires 253; found 254.0 (M+H⁺).

### Description for D28

### 5-Bromo-2-{3-chloro-4-[(1-methylethyl)oxy]phenyl}-1,3-thiazole (D28)

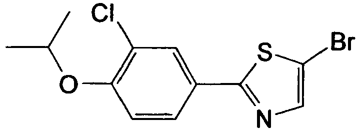

To a solution of 2-{3-chloro-4-[(1-methylethyl)oxy]phenyl}-1,3-thiazole (D27) (400 mg) and sodium acetate (259 mg) in acetic acid (10 mL) stirred at room temperature was added a solution of Br₂ (0.1 mL) in acetic acid dropwise during 30 min. The reaction mixture was stirred for another 30 min and basified with aqueous NaOH (2M), then partitioned with ethyl acetate.The organic phase was washed with water and brine, dried over sodium sulphate. After concentration, the crude product was purified by ISCO (15% ethyl acetate in hexanes) to afford 5-bromo-2-{3-chloro-4-[(1-methylethyl)oxy]phenyl}-1,3-thiazole (D28) (380 mg). δH (MeOD, 400MHz): 1.39 (6H, d), 4.74 (1H, m), 7.17 (1H, d), 7.76 (2H, m), 7.93 (1H, d). MS (ES): C₁₂H₁₁BrClNOS requires 330; found 331.0(M+H⁺).

### Description for D29

### Ethyl 3-[4-(4,4,5,5-tetramethyl-1,3,2-dioxaborolan-2-yl)-1H-pyrrolo[2,3-b]pyridin-1-yl] propanoate (D29)

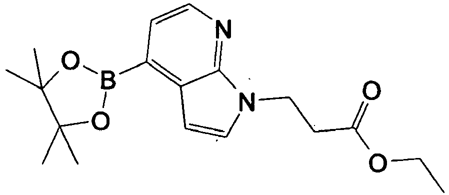

To a suspension of ethyl 3-(4-bromo-1*H*-pyrrolo[2,3-b]pyridin-1-yl)propanoate (D1) (7 g), 4,4,4',4',5,5,5',5'-octamethyl-2,2'-bi-1,3,2-dioxaborolane (17.95 g) and potassium acetate (11.56 g) in DMF (100 mL) was added PdCl₂(dppf)-CH₂Cl₂ adduct (1.154 g). The reaction mixture was degassed and charged with N₂, stirred at 85 °C for 2 h. The reaction mixture was cooled to room temperature and diluted with ethyl acetate, filtered through Celite. The filtrate was partitioned between ethyl acetate and water.The organic phase was washed with water and saturated brine, dried over sodium sulphate and evaporated in vacuo to give the crude product, which was purified by ISCO (20% ethyl acetate in hexanes) to afford ethyl 3-[4-(4,4,5,5-tetramethyl-1,3,2-dioxaborolan-2-yl)-1*H*-pyrrolo[2,3-b]pyridin-1-yl] propanoate (D29) (7.5 g). δH (CDCl₃, 400MHz): 1.21 (3H, t), 1.39 (12H, s), 2.88 (2H, t), 4.11 (2H, q), 4.61 (2H, t), 6.83 (1H, d), 7.33 (1H, d), 7.44 (1H, d), 8.32 (1H, d). MS (ES): C₁₈H₂₅BN₂O₄ requires 344; found 344.2 (M+H⁺).

### Description for D30

### Ethyl 4-[4-(4,4,5,5-tetramethyl-1,3,2-dioxaborolan-2-yl)-1H-pyrrolo[2,3-b]pyridin-1-yl] butanoate (D30)

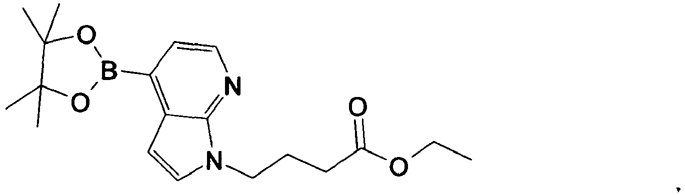

To a suspension of ethyl 4-(4-bromo-1*H*-pyrrolo[2,3-b]pyridin-1-yl)butanoate (D6) (12.5 g), 4,4,4',4',5,5,5',5'-octamethyl-2,2'-bi-1,3,2-dioxaborolane (30.6 g) and potassium acetate (19.71 g) in DMF (150 mL) was added PdCl₂(dppf)-CH₂Cl₂ adduct (1.968 g). The reaction mixture was degassed and charged with N₂, stirred at 85 °C for 2 hours. The reaction mixture was cooled to room temperature and diluted with ethyl acetate, filtered through Celite. The filtrate was partitioned between ethyl acetate and water. The organic phase was washed with water and saturated brine, dried over sodium sulphate and evaporated in vacuo to give the crude product, which was purified by ISCO (20% ethyl acetate in hexanes) to afford ethyl 4-[4-(4,4,5,5-tetramethyl-1,3,2-dioxaborolan-2-yl)-1*H*-pyrrolo[2,3-b]pyridin-1-yl] butanoate (D30) (12.5 g). δH (CDCl₃, 400MHz): 1.22 (3H, t), 1.39 (12H, s), 2.19 (2H, m), 2.28 (2H, t), 4.09 (2H, t), 4.37 (2H, q), 6.86 (1H, d), 7.27 (1H, d), 7.44 (1 H, d), 8.32 (1 H, d). MS (ES): C₁₉H₂₇BN₂O₄ requires 358; found 359.1 (M+H⁺).

### Description-for D31

### Ethyl 3-[4-(5-{3-chloro-4-[(1-methylethyl)oxy]phenyl}-1,3,4-thiadiazol-2-yl)-1H-pyrrolo[2,3-b]pyridin-1-yl]propanoate (D31)

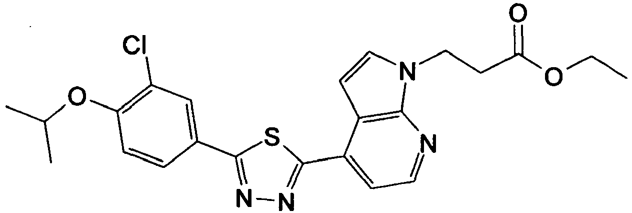

To a suspension of 2-bromo-5-{3-chloro-4-[(1-methylethyl)oxy]phenyl}-1,3,4-thiadiazole (D25) (300 mg), ethyl 3-[4-(4,4,5,5-tetramethyl-1,3,2-dioxaborolan-2-yl)-1*H*-pyrrolo[2,3-b]pyridin-1-yl] propanoate (D29) (340 mg) and Cs₂CO₃ (352 mg) in acetonitrile (6 mL)/water (2 mL) was added PdCl₂(dppf)-CH₂Cl₂ adduct (73 mg). The reaction mixture was purged with N₂, sealed in a microwave tube and stirred at 120 °C for 20 min. The reaction mixture was cooled to room temperature and diluted with ethyl acetate, filtered through Celite. The filtrate was partitioned between ethyl acetate and water. The organic phase was washed with water and saturated brine, dried over sodium sulphate and evaporated in vacuo to give the crude product ethyl 3-[4-(5-{3-chloro-4-[(1-methylethyl)oxy]phenyl}-1,3,4-thiadiazol-2-yl)-1*H*-pyrrolo[2,3-b]pyridin-1-yl]propanoate (D31) (300 mg), used directly for hydrolysis. MS (ES): C₂₃H₂₃ClN₄O₃S requires 470; found 471.2 (M+H⁺).

### Description for D32

### Ethyl 4-[4-(5-{3-chloro-4-[(1-methylethyl)oxy]phenyl}-1,3,4-thiadiazol-2-yl)-1H-pyrrolo[2,3-b]pyridin-1-yl]butanoate (D32)

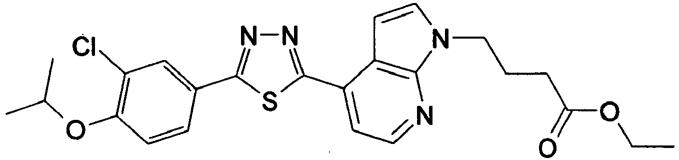

A mixture of 2-bromo-5-{3-chloro-4-[(1-methylethyl)oxy]phenyl}-1,3,4-thiadiazole (D25) (0.300 g), ethyl 4-[4-(4,4,5,5-tetramethyl-1,3,2-dioxaborolan-2-yl)-1*H-*pyrrolo[2,3-b]pyridin-1-yl]butanoate (D30) (0.322 g), tripotassium phosphate (0.477 g) and tetrakis(triphenylphosphine)palladium (0.104 g) in DMF (8 mL) and water (1.6 mL) was heated at 130 °C in a microwave reactor for 8 min, and the mixture was extracted with ethyl acetate (50 mL). The organic layers were combined, dried over sodium sulfate, and evaporated in vacuo to afford dark brown residue. The residue was purified on chromatography on silica gel to afford ethyl 4-[4-(5-{3-chloro-4-[(1-methylethyl)oxy]phenyl}-1,3,4-thiadiazol-2-yl)-1*H*-pyrrolo[2,3-b]pyridin-1-yl]butanoate (D32) (240 mg). MS (ES): C₂₄H₂₅ClN₄O3S requires 484.1; found 485.2 (M+H⁺).

### Description for D33

### Ethyl 3-[4-(5-{3-chloro-4-[(1-methylethyl)oxy]phenyl}-1,3,4-oxadiazo)-2-yl)-1iH-pyrrolo[2,3-b]pyridin-1-yl]propanoate (D33)

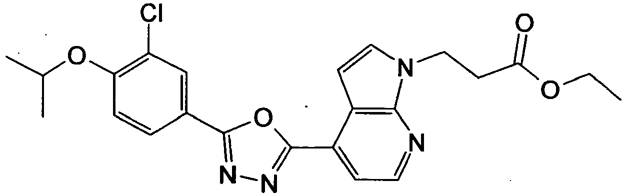

To a suspension of 2-bromo-5-{3-chloro-4-[(1-methylethyl)oxy]phenyl}-1,3,4-oxadiazole (D26) (300 mg), ethyl 3-[4-(4,4,5,5-tetramethyl-1,3,2-dioxaborolan-2-yl)-1*H*-pyrrolo[2,3-b]pyridin-1-yl] propanoate (D29) (358 mg) and Cs₂CO₃ (369 mg) in acetonitrile (6 mL)/water (2 mL) was added PdCl₂(dppf)-CH₂Cl₂ adduct (77 mg). The reaction mixture was purged with N₂, sealed in a microwave tube and stirred at 120 °C for 20 min. The reaction mixture was cooled to room temperature and diluted with ethyl acetate, filtered through Celite. The filtrate was partitioned between ethyl acetate and water. The organic phase was washed with water and saturated brine, dried over sodium sulphate and evaporated in vacuo to give the crude product ethyl 3-[4-(5-{3-chloro-4-[(1-methylethyl)oxy]phenyl}-1,3,4-oxadiazol-2-yl)-1*H*-pyrrolo[2,3-b]pyridin-1-yl]propanoate (D33) (300 mg), used directly for hydrolysis. MS (ES): C₂₃H₂₃ClN₄O₄ requires 454; found 455.2 (M+H⁺).

### Description for D34

### Ethyl 4-[4-(5-{3-chloro-4-[(1-methytethyl)oxy]pnenyl}-1,3,4-oxadiazol-2-yl)-1H-pyrrolo[2,3-b]pyridin-1-yl]butanoate (D34)

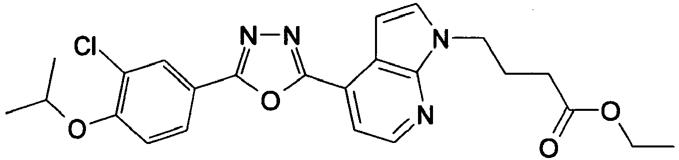

A mixture of 2-bromo-5-{3-chloro-4-[(1-methylethyl)oxy]pheny}-1,3,4-oxadiazole (D26) (0.3 g), ethyl 4-[4-(4,4,5,5-tetramethyl-1,3,2-dioxaborolan-2-yl)-1*H*-pyrrolo[2,3-b]pyridin-1-yl]butanoate (D30) (0.338 g), tripotassium phosphate (0.501 g) and tetrakis(triphenylphosphine)palladium (0.109 g) in DMF (8 mL) and water (1.6 mL) was heated at 130 °C in a microwave reactor for 8 min, and the mixture was extracted with ethyl acetate (50 mL). The organic layers were combined, dried over sodium sulfate, and evaporated in vacuo to afford dark brown residue. The residue was purified on chromatography on silica gel to afford ethyl 4-[4-(5-{3-chloro-4-[(1-methylethyl)oxy]phenyl}-1,3,4-oxadiazo-2-yl)-1*H*-pyrrolo[2,3-b]pyridin-1-yl]butanoate (D34) (300 mg). MS (ES): C₂₄H₂₅CIN₄O₄ requires 468.2; found 469.2 (M+H⁺).

### Description for D35

### Ethyl 3-[4-(2-{3-chloro-4-[(1-methylethyl)oxy]phenyl}-1,3-thiazol-5-yl)-1H-pyrrolo[2,3-b]pyridin-1-yl]propanoate (D35)

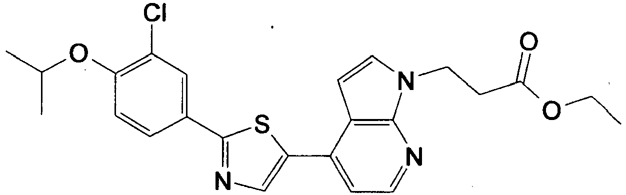

To a suspension of 5-bromo-2-{3-chloro-4-[(1-methylethyl)oxy]phenyl}-1,3-thiazole (D28) (200 mg), ethyl 3-[4-(4,4,5,5-tetramethyl-1,3,2-dioxaborolan-2-yl)-1*H-*pyrrolo[2,3-b]pyridin-1-yl] propanoate (D29) (248 mg) and Cs₂CO₃ (235 mg) in acetonitrile (6 mL)/water (1.5 mL) was added PdCl₂(dppf)-CH₂Cl₂ adduct (39 mg). The reaction mixture was purged with N₂, sealed in a microwave tube and stirred at 120 °C for 25 min. The reaction mixture was cooled to room temperature and diluted with ethyl acetate, filtered through Celite. The filtrate was partitioned between ethyl acetate and water. The organic phase was washed with water and saturated brine, dried over sodium sulphate and evaporated in vacuo to give the crude product ethyl 3-[4-(2-{3-chloro-4-[(1-methylethyl)oxy]phenyl}-1,3-thiazol-5-yl)-1*H*-pyrrolo[2,3-b] pyridin-1-yl]propanoate (D35) (300 mg), used directly for hydrolysis. MS (ES): C₂₄H₂₄CIN₃O₃S requires 469; found 470.2 (M+H⁺).

### Description for D36

### Ethyl 4-[4-(2-{3-chloro-4-[(1-methylethyl)oxy]phenyl}-1,3-thiazol-5-yl)-1H-pyrrolo[2,3-b]pyridin-1-yl]butanoate (D36)

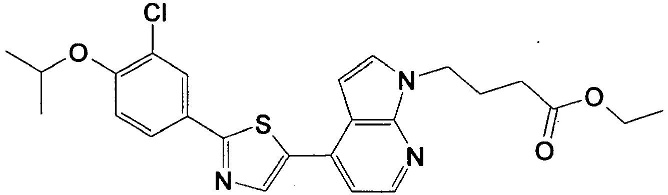

To a suspension of 5-bromo-2-{3-chloro-4-[(1-methylethyl)oxy]phenyl}-1,3-thiazole (D28) (200 mg), ethyl 4-[4-(4,4,5,5-tetramethyl-1,3,2-dioxaborolan-2-yl)-1*H* pyrrolo[2,3-b]pyridin-1-yl] butanoate (D30) (258 mg) and Cs₂CO₃ (235 mg) in acetonitrile (6 mL)/water (1.5 mL) was added PdCl₂(dppf)-CH₂Cl₂ adduct (39 mg). The reaction mixture was purged with N₂, sealed in a microwave tube and stirred at 120 °C for 25 min. The reaction mixture was cooled to room temperature and diluted with ethyl acetate, filtered through Celite. The filtrate was partitioned between ethyl acetate and water. The organic phase was washed with water and saturated brine, dried over sodium sulphate and evaporated in vacuo to give the crude product ethyl 4-[4-(2-{3-chloro-4-[(1-methylethyl)oxy]phenyl}-1,3-thiazol-5-yl)-1*H*-pyrrolo[2,3-b]pyridin-1-yl]butanoate (D36) (300 mg), used directly for hydrolysis. MS (ES): C₂₅H₂₆CIN₃O₃S requires 483; found 484.2 (M+H⁺).

### Description for D37

### 3-Chloro-4-[(1-methylethyl)oxy]benzoic acid (D37)

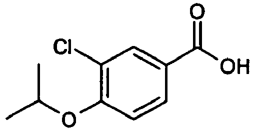

Propan-2-ol (2.45 ml) and PPh₃ (1.18 g) were dissolved in THF (30 ml), cooled to 0 °C, treated with methyl 3-chloro-4-hydroxybenzoate (6.00 g) followed by the dropwise addition of DIAD (9.44 ml) and stirred at room temperature overnight. The reaction mixture was then evaporated and purified on silica caRTridges (0 to 40 % EtOAc in pentane) to afford the crude product (7.00 g) as a colourless oil. This was dissolved in MeOH (30 ml) and 2 M aqueous NaOH (30 ml) and stirred at room temperature for a weekend. The reaction mixture was then evaporated and redissolved in H₂O. This solution was washed with Et₂O, acidified to pH = 1 and extracted with Et₂O. These latter extracts were dried over MgSO₄, filtered and evaporated to afford the title compound (D37) (4.16 g) as a white solid. δH (MeOD, 400MHz): 1.37 (6H, d), 4.77 (1H, septet), 7.12 (1 H, d), 7.90 (1 H, d), 7.98 (1H, s). MS (ES): C₁₀H₁₁ClO₃ requires 214; found 215 (MH⁺).

### Alternative synthesis:

### 3-Chloro-4-[(1-methylethyl)oxy]benzoic acid (D37)

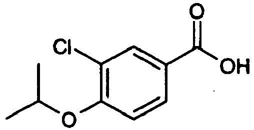

Methyl-4-hydroxy-3-chloro benzoate (13.4 g) was dissolved in DMF (150 ml), treated with K₂CO₃ (19.9 g) followed by isopropyl bromide (13.5 ml) and the resultant mixture heated to 70 °C and stirred overnight. The reaction mixture was then cooled to room temperature, evaporated to dryness, re-dissolved in EtOH, filtered and evaporated once more to afford the intermediate ester (22.2 g) as a white solid. This compound was a mixture of ethyl and methyl esters and used crude in the next reaction.

The crude intermediate (22.2 g) was dissolved in MeOH (75 ml), treated with 2M aqueous NaOH (75 ml), heated to 60 °C and stirred for 2 hours. The reaction mixture was then cooled to room temperature, the MeOH evaporated and the remaining aqueous solution acidified with 5M aqueous HCl (30 ml). The precipitate was filtered off and dried to afford the title compound (D37) (15.1 g) as a white solid. δH (CDCl₃, 400MHz): 1.42 (6H, d), 4.70 (1 H, septet), 6.97 (1H, d), 7.97 (1H, d), 8.12 (1H, s). MS (ES): C₁₀H₁₁ClO₃ requires 214; found 213 (M-H⁺).

### Description for D38

### 5-chloro-6-[(1-methylethyl)oxy]-3-pyridinecarboxylic acid (D38)

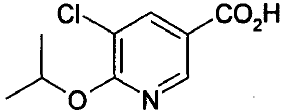

Sodium (100 g) was added to i-PrOH (5 L) with stirring in portions. After addition the resulting mixture was stirred overnight at 50 °C, and then heated to reflux for two days until residual sodium was completely consumed. The prepared solution of *i-*PrONa was cooled to 50 °C and treated portionwise with 5,6-dichloro-3-pyridinecarboxylic acid (100 g). The resulting mixture was heated at reflux for 4 hours and TLC showed the reaction completed. After cooling, it was filtered and water (1 L) was added to the filtrate. The mixture was adjusted acidified by HCl (2 N) till pH 5-6 and evaporated under reduced pressure. The precipitate was formed. The collected precipitate was dried *in vacuo* to afford 5-chloro-6-[(1-methylethyl)oxy]-3-pyridinecarboxylic acid (D38) (115 g) as a white solid. δH (CDCl₃, 400 MHz): 1.43 (6H, d), 5.47 (1 H, m), 8.23 (1 H, d), 8.75 (1H, d).

### Example 1

### 3-[4-(5-{3-chloro-4-[(1-methylethyl)oxy]phenyl}-1,2,4-oxadiazol-3-yl)-1H-pyrrolo[2,3-b]pyridin-1-yl]propanoic acid (E1)

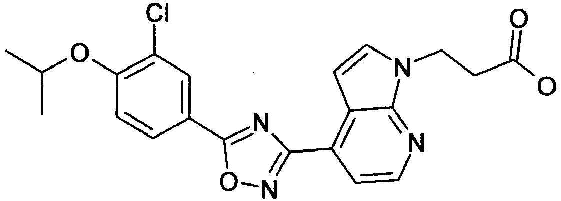

Aqueous NaOH solution (2 N, 0.2 mL) was added to a solution of 3-[4-(5-{3-chloro-4-[(1-methylethyl)oxy]phenyl}-1,2,4-oxadiazol-3-yl)-1H-pyrrolo[2,3-b]pyridin-1-yl]propanoate (D4) (134.0 mg) in isopropanol (4 mL) and H₂O (2mL) at room temperature. The reaction mixture was heated to 80 °C and stirred at that temperature for 30 min. After the reaction mixture was cooled to room temperature, the mixture was adjusted the pH under ice-bath value to about 6 and a white suspension appeared. The mixture was filtered and the filtered-cake was washed with H₂O (3mL) for 3 times. Then the filtered-cake was collected and dried to afford 3-[4-(5-{3-chloro-4-[(1-methylethyl)oxy]phenyl}-1,2,4-oxadiazol-3-yl)-1H-pyrrolo[2,3-b]pyridin-1-yl]propanoic acid (E1) as white solid (90.0 mg). δH (DMSO-*d*₆, 400MHz): 1.36 (6H, d), 2.70 (2H, t), 4.50 (2H, t), 4.88 (1H, m), 6.95 (1H, d), 7.44 (1H, d), 7.76 (1H, d), 7.80 (1H, d), 8.13 (1H, dd), 8.20 (1H, d), 8.45 (1H, d). MS (ES): C₂₁H₁₉ClN₄O₄ requires 426; found 427.1 (M+H⁺).

### Example 2

### 3-[4-(5-{5-chloro-6-[(1-methylethyl)oxy]-3-pyridinyl}-1,2,4-oxadiazol-3-yl)-1H-pyrrolo[2,3-b]pyridin-1-yl)propanoic acid (E2)

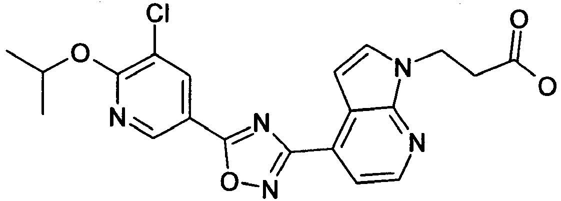

Aqueous NaOH (2N, 0.5 ml) was added to a mixture of ethyl 3-[4-(5-{5-chloro-6-[(1-methylethyl)oxy]-3-pyridinyl}-1,2,4-oxadiazol-3-yl)-1H-pyrrolo[2,3-b]pyridin-1-yl]propanoate (D5) (211.0 mg) in isopropanol (20 mL) and H₂O (10 mL) at room temperature. The reaction mixture was heated to 80 °C and stirred at that temperature for 30 min. After the mixture was adjusted the pH value to about 6 under ice-bath, the mixture was concentrated. The residue were dissolved in DMF and purified by Mass Directed AutoPrep. The solvent was evaporated in vacuo to afford 3-[4-(5-{5-chloro-6-[(1-methylethyl)oxy]-3-pyridinyl}-1,2,4-oxadiazol-3-yl)-1H-pyrrolo[2,3-b]pyridin-1-yl]propanoic acid (E2) as white solid (179.0 mg). δH (DMSO-*d*₆, 400MHz): 1.39 (6H, d), 2.87 (2H, t), 4.55 (2H, t), 5.45 (1 H, m), 7.03 (1 H, d), 7.79 (1H, d), 7.84 (1 H, d), 8.49 (1H, d), 8.60 (1H, d), 8.98 (1 H, d), 12.41 (1H, br s). MS (ES): C₂₀H₁₈CIN₅O₄ requires 427; found 428.1 (M+H⁺).

### Example 3

### 4-[4-(5-{3-chloro-4-[(1-methylethyl)oxy]phenyl}-1,2,4-oxadiazol-3-yl)-1H-pyrrolo[2,3-b]pyridin-1-yl]butanoic acid (E3)

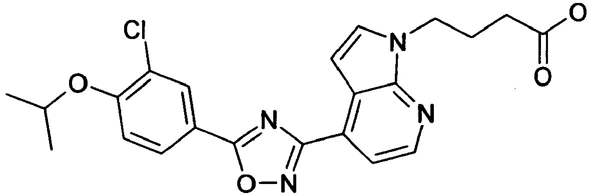

Aqueous NaOH solution (2 N, 0.4 mL) was added to a mixture of ethyl 4-[4-(5-{3-chloro-4-[(1-methylethyl)oxy]phenyl}-1,2,4-oxadiazol-3-yl)-1H-pyrrolo[2,3-b]pyridin-1-yl]butanoate (D9) (181.0 mg) in isopropanol (20 mL) and H₂O (5mL) at room temperature. The reaction mixture was heated to 80 °C and stirred at that temperature for 30 min. After the mixture was adjusted the pH value to about 6 under ice-bath, the mixture was concentrated. The residue were dissolved in DMF and purified by Mass Directed AutoPrep. The solvent was evaporated in vacuo to afford 4-[4-(5-{3-chloro-4-[(1-methylethyl)oxy]phenyl}-1,2,4-oxadiazol-3-yl)-1H-pyrrolo[2,3-b]pyridin-1-yl]butanoic acid (E3) as white solid (153.0 mg). δH (DMSO-*d*₆, 400MHz): 1.36 (6H, d), 2.06 (2H, m), 2.21 (2H, t), 4.37 (2H, t), 4.89 (1H, m), 7.03 (1 H, d), 7.46 (1H, d), 7.80 (1 H, d), 7.83 (1 H, d), 8.16 (1H, dd), 8.23 (1 H, d), 8.47 (1 H, d), 12.13 (1 H, br s). MS (ES): C₂₂H₂₁CIN₄O₄ requires 440; found 441.2 (M+H⁺).

### Example 4

### 4-[4-(5-{5-chloro-6-[(1 methylethyl)oxy]-3-pyridinyl}-1,2,4-oxadiazol-3-yl)-1H-pyrrolo[2,3-b]pyridin-1-yl]butanoic acid (E4)

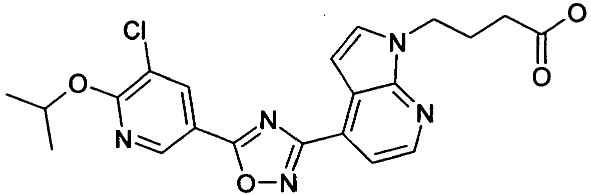

Aqueous NaOH (2N, 0.5 ml) was added to a mixture of ethyl 4-[4-(5-{5-chloro-6-[(1-methylethyl)oxy]-3-pyridinyl}-1,2,4-oxadiazol-3-yl)-1*H*-pyrrolo[2,3-*b*]pyridin-1-yl]butanoate (D10) (256.0 mg) in isopropanol (20 mL) and H₂O (10 mL) at room temperature. The reaction mixture was heated to 80 °C and stirred at that temperature for 30 min. After the mixture was adjusted the pH value to about 6 under ice-bath, the mixture was concentrated. The residue were dissolved in DMF and purified by Mass Directed AutoPrep. The solvent was evaporated in vacuo to afford 4-[4-(5-{5-chloro-6-[(1-methylethyl)oxy]-3-pyridinyl}-1,2,4-oxadiazol-3-yl)-1*H-*pyrrolo[2,3-*b*]pyridin-1-yl]butanoic acid (E4) as white solid (166.0 mg). δH (DMSO-*d*₆, 400MHz): 1.39 (6H, d), 2.07 (2H, m), 2.20 (2H, t), 4.37 (2H, t), 5.45 (1 H, m), 7.05 (1 H, d), 7.81 (1 H, d), 7.83 (1 H, d), 8.48 (1 H, d), 8.61 (1 H, d), 8.98 (1 H, d), 12.13 (1 H, br s). MS (ES): C₂₁H₂₀CIN₅O₄ requires 441; found 442.1 (M+H⁺).

### Example 5

### 3-[4-(5-{3-chloro-4-[(1-methylethyl)oxy]phenyl}-1,2,4-oxadiazol-3-yl)-1H-pyrrolo[2,3-c]pyridin-1-yl]propanoic acid (E5)

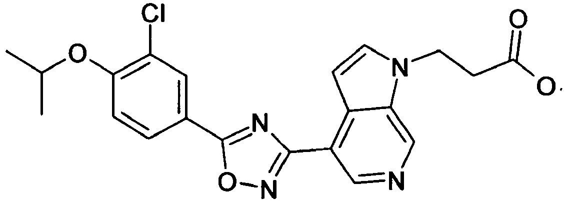

Aqueous NaOH (2N, 0.5mL) was added to a mixture of ethyl 3-[4-(5-{3-chloro-4-[(1-methylethyl)oxy]phenyl}-1,2,4-oxadiazol-3-yl)-1H-pyrrolo[2,3-c]pyridin-1-yl]propanoate (D20) (288.0 mg) in isopropanol (40 mL) and H₂O (5 mL) at room temperature. After the reaction mixture was heated to 80°C for 1h, the mixture was concentrated to remove most of isopropanol, adjusted pH value to 6 under ice-bath and and a white suspension appeared. The mixture was filtered and the filtered-cake was washed with H₂O (2 mL) for 3 time, dried and recrystalized via acetronitrile (250 mL) to afford 3-[4-(5-{3-chloro-4-[(1-methylethyl)oxy]phenyl}-1,2,4-oxadiazol-3-yl)-1H-pyrrolo[2,3-c]pyridin-1-yl]propanoic acid (E5) as white solid (180.0 mg). δH (DMSO-*d*₆, 400MHz): 1.36 (6H, d), 2.87 (2H, t), 4.61 (2H, t), 4.89 (1 H, m), 7.03 (1H, d), 7.46 (1 H, d), 7.84 (1H, d), 8.16 (1H, dd), 8.24 (1 H, d), 8.93 (1H, s), 9.13 (1 H, s), 12.43 (1 H, br s). MS (ES): C₂₁H₁₉ClN₄O₄ requires 426; found 427.2 (M+H⁺).

### Example 6

### 4-[4-(5-{3-chloro-4-[(1-methylethyl)oxy]phenyl)-1,2,4-oxadiazol-3-yl)-1H-pyrrolo[2,3-c]pyridin-1-yl]butanoic acid (E6)

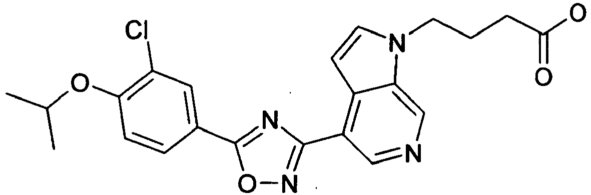

Aqueous NaOH (2N, 0.3 mL) was added to a mixture of ethyl 4-[4-(5-{3-chloro-4-[(1-methylethyl)oxy]phenyl}-1,2,4-oxadiazol-3-yl)-1H-pyrrolo[2,3-c]pyridin-1-yl]butanoate (D21) (180.0 mg) in isopropanol (40 mL) and H₂O (5 mL) at room temperature. After the reaction mixture was heated to 80°C for 1h, the mixture was concentrated to remove most of isopropanol, adjusted pH value to 6 under ice-bath and a brown suspension appeared. The mixture was filtered and the filtered-cake was washed with H₂O (2 mL) for 3 time, dried to afford 4-[4-(5-{3-chloro-4-[(1-methylethyl)oxy]phenyl}-1,2,4-oxadiazol-3-yl)-1H-pyrrolo[2,3-c]pyridin-1-yl]butanoic acid (E6) (100.0 mg) as brown solid. δH (DMSO-*d*₆, 400MHz): 1.36 (6H, d), 2.06 (2H, m), 2.23 (2H, t), 4.42 (2H, t), 4.89 (1H, m), 7.05 (1H, d), 7.46 (1 H, d), 7.85 (1 H, d), 8.16 (1H, dd), 8.24 (1H, d), 8.94 (1H, d), 9.11 (1H, d), 12.18 (1H, br s). MS (ES): C₂₂H₂₁CIN₄O₄ requires 440; found 441.2 (M+H⁺).

### Example 7

### 3-[4-(5-{5-chloro-6-[(1-methylethyl)oxy]-3-pyridinyl}-1,2,4-oxadiazol-3-yl)-1H-pyrrolo[2,3-c]pyridin-1-yl]propanoic acid (E7)

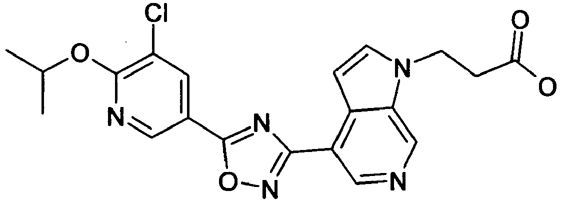

Aqueous NaOH (2N, 0.6 mL) was added to a mixture of ethyl 3-[4-(5-{5-chloro-6-[(1-methylethyl)oxy]-3-pyridinyl}-1,2,4-oxadiazol-3-yl)-1H-pyrrolo[2,3-c]pyridin-1-yl]propanoate (D23) (331.0 mg) in isopropanol (40 mL) and H₂O (5 mL) at room temperature. After the reaction mixture was heated to 80°C for 1h, the mixture was concentrated to remove most of isopropanol (40 mL), adjusted the pH value to 6 under ice-bath and a white suspension appeared. The mixture was filtered, the filtered-cake was washed with H₂O (2 mL) for 3 times and dried to afford 3-[4-(5-{5-chloro-6-[(1-methylethyl)oxy]-3-pyridinyl}-1,2,4-oxadiazol-3-yl)-1H-pyrrolo[2,3-c]pyridin-1-yl]propanoic acid (E7) as white solid (210.0 mg). δH (DMSO-*d*₆, 400MHz): 1.32 (6H, d), 2.80 (2H, t), 4.54 (2H, t), 5.38 (1H, m), 6.97 (1 H, d), 7.78 (1H, d), 8.54 (1 H, d), 8.87 (1H, s), 8.91 (1 H, d), 9.06 (1 H, s), 12.37 (1 H, br s). MS (ES): C₂₀H₁₈CIN₅O₄ requires 427; found 428.2 (M+H⁺).

### Example 8

### 4-[4-(5-(5-chloro-6-[(1-methylethyl)oxy]-3-pyridinyl}-1,2,4-oxadiazol-3-yl)-1H-pyrrolo[2,3-c]pyridin-1-yl]butanoic acid (E8)

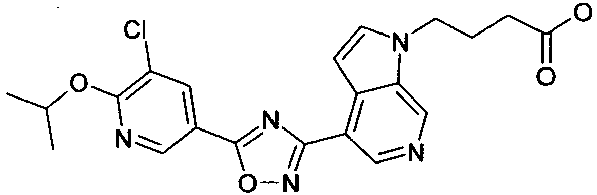

Aqeous NaOH (2N 0.5 mL) was added to a mixture of ethyl 4-[4-(5-{5-chloro-6-[(1-methylethyl)oxy]-3-pyridinyl}-1,2,4-oxadiazol-3-yl)-1H-pyrrolo[2,3-c]pyridin-1-yl]butanoate (D24) (290.0 mg) in isopropanol (40 mL) and H₂O (10 mL) at room temperature. After the reaction mixture was heated to 80°C for 1h, the mixture was concentrated and removed most of the isopropanol (40 mL), adjusted pH value to 6 under ice-bath and a gray suspension was appeared. The mixture was filtered, the filtered-cake was washed with H₂O (2 mL) for 3 times and dried to afford 4-[4-(5-{5-chloro-6-[(1-methylethyl)oxy]-3-pyridinyl}-1,2,4-oxadiazol-3-yl)-1H-pyrrolo[2,3-c]pyridin-1-yl]butanoic acid (E8) as brown solid (226.0 mg). δH (DMSO-*d*₆, 400MHz): 1.46 (6H, d), 2.12 (2H, m), 2.30 (2H, t), 4.48 (2H, t), 5.52 (1H, m), 7.12 (1 H, d), 7.92 (1 H, d), 8.66 (1 H, d), 9.00 (1H, s), 9.03 (1 H, d), 9.17 (1H, s), 12.28 (1H, br s). MS (ES): C₂₁H₂₀CIN₅O₄ requires 441; found 442.1 (M+H⁺).

### Example 9

### 3-[4-(5-{3-Chloro-4-[(1-methylethyl)oxy]phenyl}-1,3,4-thiadiazol-2-yl)-1H-pyrrolo[2,3-b]pyridin-1-yl]propanoic acid (E9)

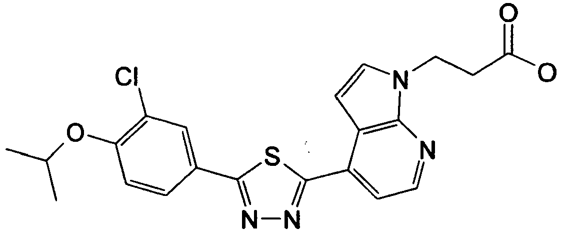

To a solution of ethyl 3-[4-(5-{3-chloro-4-[(1-methylethyl)oxy]phenyl}-1,3,4-thiadiazol-2-yl)-1*H*-pyrrolo[2,3-b]pyridin-1-yl]propanoate (D31) (200 mg) in DMF (5 mL) was added aqueous NaOH (2M, 1 mL). The reaction mixture was stirred at 50°C for 2 hours. The reaction mixture was acidified with aqueous HCl (2M) to pH 3-4, partitioned between ethyl acetate and water. The organic phase was washed with water and saturated brine, dried over sodium sulphate and evaporated in vacuo to give the crude product, which was purified by MDAP to afford 3-[4-(5-{3-chloro-4-[(1-methylethyl)oxy]phenyl}-1,3,4-thiadiazol-2-yl)-1*H*-pyrrolo[2,3-b]pyridin-1-yl]propanoic acid (E9) (70 mg). δH (DMSO-*d*₆, 400MHz): 1.29 (6H, d), 2.81 (2H, t), 4.50 (2H, t), 4.79 (1H, m), 6.99 (1H, d), 7.34 (1 H, d), 7.66 (1 H, d), 7.77 (1H, d), 7.97 (1H, dd), 8.09 (1H, d), 8.39 (1H, d), 12.36 (1H, s). MS (ES): C₂₁H₁₉CIN₄O₃S requires 442 ; found 443.1 (M+H⁺).

### Example 10

### 4-[4-(5-{3-Chloro-4-[(1-methylethyl)oxy]phenyl}-1,3,4-thiadiazol-2-yl)-1H-pyrrolo[2,3-b]pyridin-1-yl]butanoic acid (E10)

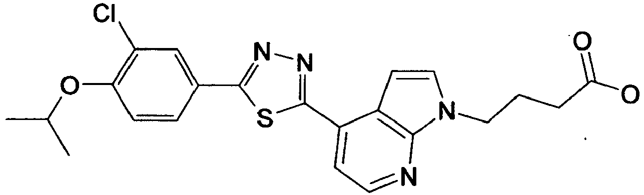

To a mixture of ethyl 4-[4-(5-{3-chloro-4-[(1-methylethyl)oxy]phenyl}-1,3,4-thiadiazol-2-yl)-1*H*-pyrrolo[2,3-b]pyridin-1-yl]butanoate (D32) (240 mg) in isopropanol (2.5 mL) and water (2.5 mL) was added lithium hydroxide (41.5 mg),. The mixture was stirred at 79 °C for 2 hours and neutralized with AcOH and evaporated under high-vacuum, dissolved in DMF for MDAP to obtain 4-[4-(5-{3-chloro-4-[(1-methylethyl)oxy]phenyl}-1,3,4-thiadiazol-2-yl)-1*H*-pyrrolo[2,3-b]pyridin-1-yl]butanoic acid (E10) (70 mg). δH (OMSO-*d*₆, 400 MHz): 1.32 (6H, d), 2.08 (2H, m), 2.21 (2H, t), 4.37 (2H, t), 4.84 (1 H, m), 7.07 (1H, br s), 7.38 (1 H, d), 7.70 (1 H, d), 7.84 (1 H, br s), 7.99 (1 H, d), 8.13 (1 H, brs), 8.43 (1 H, d). MS (ES): C₂₂H₂₁CIN₄O₃S requires 456.1; found 457.1 (M+H⁺).

### Example 11

### 3-[4-(5-{3-Chloro-4-[(1-methylethyl)oxy]phenyl}-1,3,4-oxadiazol-2-yl)-1H-pyrrolo[2,3-b]pyridin-1-yl]propanoic acid (E11)

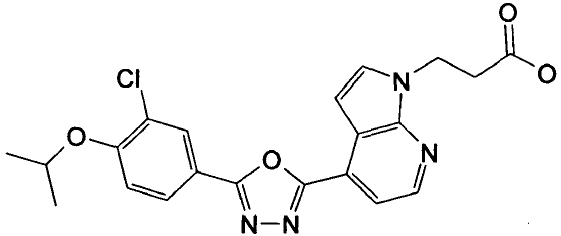

To a solution of ethyl 3-[4-(5-{3-chloro-4-[(1-methylethyl)oxy]phenyl}-1,3,4-oxadiazol-2-yl)-1*H*-pyrrolo[2,3-b]pyridin-1-yl]propanoate (D33) (200 mg) in DMF (5 mL) was added aqueous NaOH (2M, 1 mL). The reaction mixture was stirred at 50°C for 2 hours. The reaction mixture was acidified with aqueous HCl (2M) to pH 3-4, partitioned between ethyl acetate and water. The organic phase was washed with water and saturated brine, dried over sodium sulphate and evaporated in vacuo to give the crude product, which was purified by MDAP to afford 3-[4-(5-{3-chloro-4-[(1-methylethyl)oxy]phenyl}-1,3,4-oxadiazol-2-yl)-1*H*-pyrrolo[2,3-b]pyridin-1-yl]propanoic acid (E11) (130 mg). δH (CDCl₃, 400MHz): 1.46 (6H, d), 3.04 (2H, t), 4.71 (3H, m), 7.09 (1 H, d), 7.20 (1 H, d), 7.52 (1 H, d), 7.81 (1 H, d), 8.06 (1 H, dd), 8.19 (1H, d), 8.48 (1H, d). MS (ES): C₂₁H₁₉CIN₄O₄ requires 426 ; found 427.2 (M+H⁺).

### Example 12

### 4-[4-(5-{3-Chloro-4-[(1-methylethyl)oxy]phenyl}-1,3,4-oxadiazol-2-yl)-1H-pyrrolo[2,3-b]pyridin-1-yl]butanoic acid (E12)

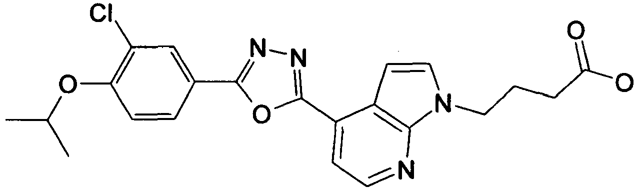

To a mixture of ethyl 4-[4-(5-{3-chloro-4-[(1-methylethyl)oxy]phenyl}-1,3,4-oxadiazol-2-yl)-1*H-*pyrrolo[2,3-b]pyridin-1-yl]butanoate (D34) (300 mg) in isopropanol (2.5 mL) and water (2.5 mL) was added lithium hydroxide (53.7 mg). The mixture was stirred at 79 °C for 2 hours and neutralized with AcOH and evaported under high-vacuum, dissolved in DMF for MDAP to obtain 4-[4-(5-{3-chloro-4-[(1-methylethyl)oxy]phenyl}-1,3,4-oxadiazol-2-yl)-1*H*-pyrrolo[2,3-b]pyridin-1-yl]butanoic acid (E12) (170 mg). δH (DMSO-*d*₆, 400 MHz): 1.36 (6H, d), 2.08 (2H, m), 2.22 (2H, t), 4.39 (2H, t), 4.87 (1H, m), 7.10 (1 H, d), 7.44 (1 H, d), 7.87 (1H, m), 8.11 (1H, dd), 8.21 (1 H, d), 8.48 (1 H, d). MS (ES): C₂₂H₂₁CIN₄O₄ requires 440.1; found 441.2 (M+H⁺).

### Example 13

### 3-[4-(2-{3-Chloro-4-[(1-methylethyl)oxy]phenyl}-1,3-thiazol-5-yl)-1H-pyrrolo[2,3-b]pyridin-1-yl]propanoic acid (E13)

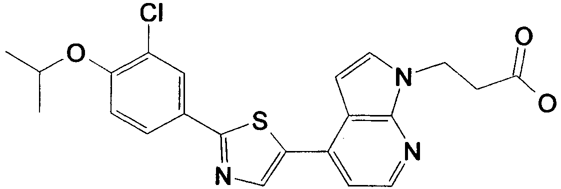

To a solution of ethyl 3-[4-(2-{3-chloro-4-[(1-methylethyl)oxy]phenyl}-1,3-thiazol-5-yl)-1H-pyrrolo[2,3-b] pyridin-1-yl]propanoate (D35) (200 mg) in DMF (5 mL) was added aqueous NaOH (2M, 1 mL). The reaction mixture was stirred at 50 °C for 2 hours. The reaction mixture was acidified with aqueous HCl (2M) to pH 3-4, partitioned between ethyl acetate and water. The organic phase was washed with water and saturated brine, dried over sodium sulphate and evaporated in vacuo to give the crude product, which was purified by MDAP to afford 3-[4-(2-{3-chloro-4-[(1-methylethyl)oxy]phenyl}-1,3-thiazol-5-yl)-1*H*-pyrrolo[2,3-b] pyridin-1-yl]propanoic acid (E13) (130 mg). δH (DMSO-*d*₆, 400MHz): 1.28 (6H, d), 2.79 (2H, t), 4.46 (2H, t), 4.75 (1 H, m), 6.83 (1 H, d), 7.28 (1 H, d), 7.41 (1 H, d), 7.65 (1H, d), 7.89 (1 H, dd), 8.00 (1 H, d), 8.26 (1H, d), 8.51 (1 H, s), 12.34 (1 H, s). MS (ES): C₂₂H₂₀CIN₃O₃S requires 441 ; found 442.2 (M+H⁺).

### Example 14

### 4-[4-(2-{3-chloro-4-[(1-methylethyl)oxy]phenyl}-1,3-thiazol-5-yl)-1H-pyrrolo[2,3-b]pyridin-1-yl]butanoic acid (E14)

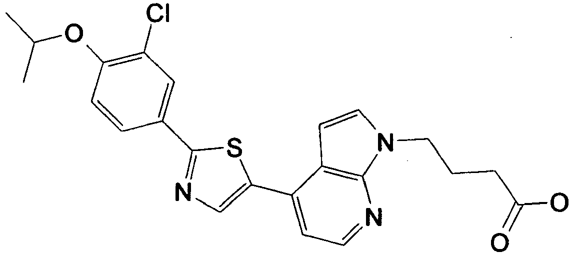

To a solution of ethyl 4-[4-(2-{3-chloro-4-[(1-methylethyl)oxy]phenyl}-1,3-thiazol-5-yl)-1H-pyrrolo[2,3-b] pyridin-1-yl]butanoate (D36) (240 mg) in DMF (5 mL) was added aqueous NaOH (2M, 1 mL). The reaction mixture was stirred at 50 °C for 2 hours.

The reaction mixture was acidified with aqueous HCl (2M) to pH 3-4, partitioned between ethyl acetate and water. The organic phase was washed with water and saturated brine, dried over sodium sulphate and evaporated in vacuo to give the crude product, which was purified by MDAP to afford 4-[4-(2-{3-chloro-4-[(1-methylethyl)oxy]phenyl}-1,3-thiazol-5-yl)-1*H*-pyrrolo[2,3-b] pyridin-1-yl]butanoic acid (E14) (210 mg) as a TFA salt. δH (DMSO-*d*₆, 400MHz): 1.28 (6H, d), 1.99 (2H, m), 2.14 (2H, t), 4.27 (2H, t), 4.75 (1 H, m), 6.86 (1 H, d), 7.28 (1 H, d), 7.40 (1H, d), 7.68 (1H, d), 7.89 (1H, dd), 8.00 (1H, d), 8.26 (1H, d), 8.52 (1H, s). MS (ES): C₂₃H₂₂CIN₃O₃S requires 455 ; found 456.2 (M+H⁺).

### S1P1 Tango assay - 96 well format

Recombinant EDG1-bla/U2OS cells (contain the human Endothelial Differentiation Gene 1 (EDG1) linked to a TEV protease site and a Gal4-VP16 transcription factor stably integrated into the Tango GPCR-bla U2OS parental cell line) were suspended in assay medium (Invitrogen Freestyle Expression Medium) at a density of 312, 500 cells/ml. Add 100µl/well of the assay medium to the cell-free control wells (column 12) and 100µl/well of the cell suspension to the test compound wells (row 2-8, column 1-10), the unstimulated control wells (DMSO) (column 11), and stimulated control wells (S1P) (row 1, column 1-10) in a Corning black-well, clear bottom 96-well plate. Cells were incubated at 37°C, 5% CO₂ for 44-48h.

Add 25µl of 5× stock solution of test compounds in assay medium with 0.5% DMSO to the test compound wells, 25µl of 5× stock solution of agonist (S1P) in assay medium with 0.5% DMSO to the stimulated compound wells, and 25µl of 5× stock solution of 0.5% DMSO in assay medium to the unstimulated control and cell-free control wells.

After incubation at 37°C, 5% CO₂ for 5h, 25µl of 6× substrate mixture (6µl Solution A (1mg LiveBLAzer™-FRET B/G substrate (CCF4-AM) in 912µl DMSO) plus 60µl Solution B plus 934µl Solution C) was added to each well and incubate at room temperature for 2h in dark. The plate was finally read on EnVision for two emission channels (460 nm and 530 nm).

All test compounds were dissolved in DMSO at a concentration of 10mM and were prepared in 100% DMSO using a 1 in 5 dilution step to provide 10 point dose response curves. The dilutions were transferred to the assay plates ensuring that the DMSO concentration was constant across the plate for all assays.

Calculate the blue/green emission ratio (460 nm/530 nm) for each well, by dividing the background-subtracted Blue emission values by the background-subtracted Green emission values. The dose response curve is based on sigmoidal dose-response model. All ratio data was normalized based upon the maximum emission ratio of positive control (S1P) and minimum emission ratio of negative control (DMSO) on each plate. The intrinsic activity (IA) of each compound would be the normalized percentage of its maximum response after curve fitting.

Exemplified compounds of the invention had a pEC50 > 7.5.

### S1 P1 Tango assay - 384 well format

Recombinant EDG1-bla/U2OS cells (contain the human Endothelial Differentiation Gene 1 (EDG1) linked to a TEV protease site and a Gal4-VP16 transcription factor stably integrated into the Tango GPCR-bla U2OS parental cell line) were harvested from growth medium and passaged into assay medium (Invitrogen Freestyle Expression Medium). The cells were starved for 24 hours at 37°C, 5% CO₂, harvested and resuspended in assay medium at a density of -200,000 cells/ml.

All test compounds were dissolved in DMSO at a concentration of 10mM and were prepared in 100% DMSO to provide 10 point dose response curves. Test compounds prepared by Bravo (Velocity11) were added to wells in columns 2-11 and 13-22; DMSO was added to wells in columns 12 and 23 as unstimulated controls and assay medium was added to wells in columns 1 and 24 as cell-free controls. An S1P1 agonist was added to wells in row 2, columns 2-11 as stimulated controls and test compounds were added to wells in row 2, columns 13-22 and rows 3-15, columns 2-11/13-22 (row 1 and 16 were empty and not used). Compounds in solution were added to the assay ptate (Greiner 781090) using an Echo (Labcyte) dose-response program (50nl/well). The unstimulated and cell-free controls were loaded with 50nl/well pure DMSO to ensure that the DMSO concentration was constant across the plate for all assays. 50 µl of the cell suspension was added to each well in columns 2-23 of the plate (∼10,000 cells per well). 50 µl of assay medium was added to each well in the cell-free controls (columns 1 and 24). The cells were incubated overnight at 37°C/5% CO₂.

10µl of 6x substrate mixture (LiveBLAzer™-FRET B/G substrate (CCF4-AM) Cat # K1096 from Invitrogen, Inc.) was added to each well using Bravo and the plates incubated at room temperature for 2h in the dark. The plate was finally read on EnVision using one excitation channel (409 nm) and two emission channels (460 nm and 530 nm).

The blue/green emission ratio (460 nm/530 nm) was calculated for each well, by dividing the background-subtracted Blue emission values by the background-subtracted Green emission values. The dose response curve is based on sigmoidal dose-response model. All ratio data was normalized based upon the maximum emission ratio of positive control and minimum emission ratio of negative control (DMSO) on each plate. The intrinsic activity (IA) of each compound would be the normalized percentage of its maximum response after curve fitting.

Exemplified compounds of the invention had a pEC50 > 7.5.

### S1 P3 GeneBlazer assay

GeneBLAzer EDG3-Ga15-NFAT-bla HEK 293T cells (contain the human Endothelial Differentiation G-protein Coupled Receptor 3 (EDG3) and a beta-lactamase reporter gene under control of a NFAT response element and a promiscuous G Protein, Ga15, stably integrated into the GeneBLAzer Ga15-NFAT-bla HEK 293T cell line) were suspended in assay medium (99% DMEM, 1% Dialyzed FBS, 0.1mM NEAA, 25mM HEPES (pH 7.3), 100U/ml penicillin, 100µg/ml streptomycin) at a density of 312, 500 cells/ml. Add 100µl/well of the assay medium to the cell-free control wells (column 12) and 100µl/well of the cell suspension to the test compound wells (row 2-8, column 1-10), the unstimulated control wells (DMSO) (column 11), and stimulated control wells (S1P) (row 1, column 1-10) in a Corning black-well, clear bottom 96-well plate. Cells were incubated at 37°C, 5% CO2 for 24h.

Add 25µl of 5× stock solution of test compounds in assay medium with 0.5% DMSO to the test compound wells, 25µl of 5× stock solution of agonist (S1P) in assay medium with 0.5% DMSO to the stimulated compound wells, and 25µl of 5× stock solution of 0.5% DMSO in assay medium to the unstimulated control and cell-free Control wells.

After incubation at 37°C, 5% CO2 for 5h, 25µl of 6× substrate mixture (6µl Solution A (1 mg LiveBLAzer™-FRET B/G Substrate (CCF4-AM) in 912µl DMSO) plus 60µl Solution B plus 934µl Solution C) was added to each well and incubate at room temperature for 2h in dark. The plate was finally read on EnVision for two emission channels (460 nm and 530 nm).

All test compounds were dissolved in DMSO at a concentration of 10mM and were prepared in 100% DMSO using a 1 in 5 dilution step to provide 10 point dose response curves. The dilutions were transferred to the assay plates ensuring that the DMSO concentration was constant across the plate for all assays.

Calculate the blue/green emission ratio (460 nm/530 nm) for each well, by dividing the background-subtracted Blue emission values by the background-subtracted green emission values. The dose response curve is based on sigmoidal dose-response model. All ratio data was normalized based upon the maximum emission ratio of positive control (S1P) and minimum emission ratio of negative control (DMSO) on each plate. The intrinsic activity (IA) of each compound would be the normalized percentage of its maximum response after curve fitting.

Exemplified compounds of the invention tested in at least one of the above assays had a pEC50 < 6.

## Claims

1. A compound of formula (I) or a salt thereof: 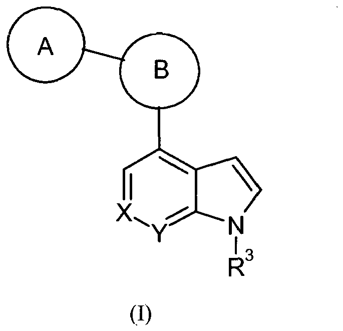 wherein
A is an aromatic ring selected from: 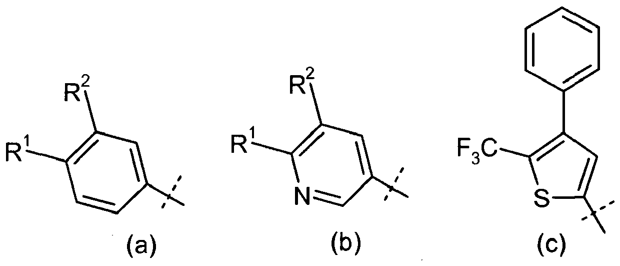 R¹ is C₍₁₋₆₎alkoxy or C₍₁₋₆₎alkyl;
R² is halogen, cyano or CF₃;
R³ is Z-COOH or C₍₁₋₆₎alkylOH;
B is a 5-membered heteroaryl ring selected from: 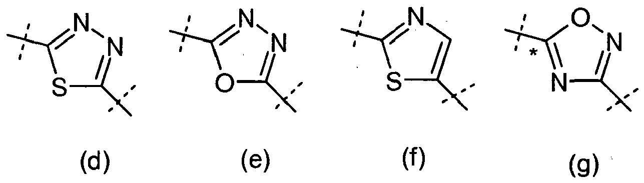 * indicates the point of attachment to the A ring;
Z is C₍₁₋₆₎alkyl or C₍₂₋₆₎alkenyl;
when Z is C₍₁₋₆₎alkyl it is optionally interrupted by a cyclopropyl, piperidinyl, azetidinyl, pyrrolidinyl, optionally interrupted by N or O and optionally substituted by O, cyclopropyl, halogen or methyl, when Z is C₍₂₋₆₎alkenyl it is optionally substituted by methyl; and
one of X and Y is CH and the other is N.

2. A compound of formula (I) according to claim 1 or a salt thereof, wherein:
R¹ is C₍₁₋₃₎alkoxy;
R² is cyano or chloro;
R³ is Z-COOH; and
Z is C₍₂₋₃₎alkyl.
A is (a) or (b);
B is (d), (f) or (g): and
one of X and Y is CH and the other N.

3. A compound of formula (I) according to claim 1 selected from:
3-[4-(5-{3-chloro-4-[(1-methylethyl)oxy]phenyl}-1,2,4-oxadiazol-3-yl)-1*H*-pyrrolo[2,3-*b*]pyridin-1-yl]propanoic acid
3-[4-(5-{5-chloro-6-[(1-methylethyl)oxy]-3-pyridinyl}-1,2,4-oxadiazol-3-yl)-1H-pyrrolo[2,3-b]pyridin-1-yl]propanoic acid
4-[4-(5-{3-chloro-4-[(1-methylethyl)oxy]phenyl}-1,2,4-oxadiazol-3-yl)-1H-pyrrolo[2,3-b]pyridin-1-yl]butanoic acid
4-[4-(5-{5-chloro-6-[(1-methylethyl)oxy]-3-pyridinyl}-1,2,4-oxadiazol-3-yl)-1*H*-pyrrolo[2,3-*b*]pyridin-1-yl]butanoic acid
3-[4-(5-{3-chloro-4-[(1-methylethyl)oxy]phenyl}-1,2,4-oxadiazol-3-yl)-1*H*-pyrrolo[2,3-*c*]pyridin-1-yl]propanoic acid
4-[4-(5-{3-chloro-4-[(1-methylethyl)oxy]phenyl}-1,2,4-oxadiazol-3-yl)-1*H*-pyrrolo[2,3-*c*]pyridin-1-yl]butanoic acid
3-[4-(5-{5-chloro-6-[(1-methylethyl)oxy]-3-pyridinyl}-1,2,4-oxadiazol-3-yl)-1*H*-pyrrolo[2,3-*c*]pyridin-1-yl]propanoic acid
4-[4-(5-{5-chloro-6-[(1-methylethyl)oxy]-3-pyridinyl}-1,2,4-oxadiazol-3-yl)-1*H*-pyrrolo[2,3-*c*]pyridin-1-yl]butanoic acid
3-[4-(5-{3-Chloro-4-[(1-methylethyl)oxy]phenyl}-1,3,4-thiadiazol-2-yl)-1*H*-pyrrolo[2,3-b]pyridin-1-yl]propanoic acid
4-[4-(5-{3-Chloro-4-[(1-methylethyl)oxy]phenyl}-1,3,4-thiadiazol-2-yl)-1*H*-pyrrolo[2,3-b]pyridin-1-yl]butanoic acid
3-[4-(5-{3-Chloro-4-[(1-methylethyl)oxy]phenyl}-1,3,4-oxadiazol-2-yl)-1*H*- pyrrolo[2,3-b]pyridin-1-yl]propanoic acid
4-[4-(5-{3-Chloro-4-[(1-methylethyl)oxy]phenyl}-1,3,4-oxadiazol-2-yl)-1*H*-pyrrolo[2,3-b]pyridin-1-yl]butanoic acid
3-[4-(2-{3-Chloro-4-[(1-methylethyl)oxy]phenyl}-1,3-thiazol-5-yl)-1*H*-pyrrolo[2,3-b]pyridin-1-yl]propanoic acid
4-[4-(2-{3-chloro-4-[(1-methylethyl)oxy]phenyl}-1,3-thiazol-5-yl)-1*H*-pyrrolo[2,3-b]pyridin-1-yl]butanoic acid
and salts thereof.

4. Use of a compound according to any one of claims 1 to 3 to manufacture a medicament for use in the treatment of conditions or disorders mediated by S1P1 receptors.

5. Use according to claim 4, wherein the condition or disorder multiple sclerosis, autoimmune diseases, chronic inflammatory disorders, asthma, inflammatory neuropathies, arthritis, transplantation, Crohn's disease, ulcerative colitis, lupus erythematosis, psoriasis, ischemia-reperfusion injury, solid tumours, and tumour metastasis, diseases associated with angiogenesis, vascular diseases, pain conditions, acute viral diseases, inflammatory bowel conditions, insulin and non-insulin dependant diabetes.

6. Use according to claim 5, wherein the condition is multiple sclerosis.

7. A pharmaceutical composition comprising a compound of formula (I) or a pharmaceutically acceptable salt thereof according to any one of claims 1 to 3.

## Patentansprüche

1. Eine Verbindung der Formel (I) oder ein Salz davon: 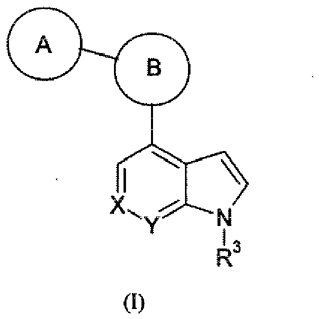 wobei
A ein aromatischer Ring ist, ausgewählt aus: 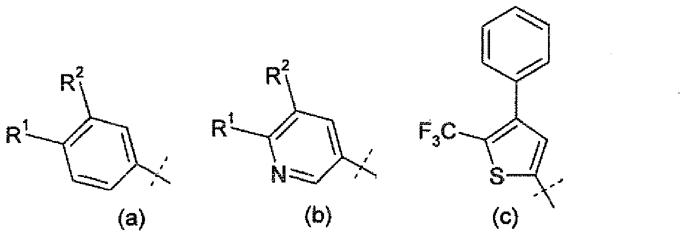 R¹ C₍₁₋₆₎-Alkoxy oder C₍₁₋₆₎-Alkyl ist;
R² Halogen, Cyano oder CF₃ ist;
R³ Z-COOH oder C₍₁₋₆₎-AlkylOH ist;
B ei 5-gliedriger Heteroarylring ist, ausgewählt aus: 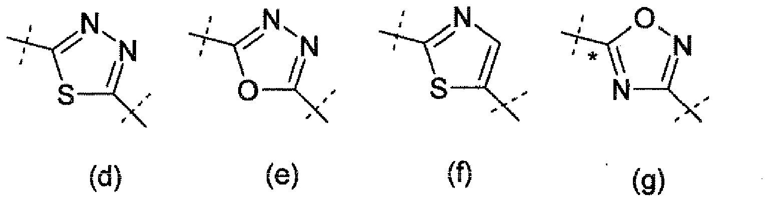 * den Ort der Bindung an den A-Ring angibt;
Z C₍₁₋₆₎-Alkyl oder C₍₂₋₆₎-Alkenyl ist;
wenn Z C₍₁₋₆₎-Alkyl ist, es gegebenenfalls durch ein Cyclopropyl, Piperidinyl, Azetidinyl, Pyrrolidinyl unterbrochen ist, gegebenenfalls unterbrochen durch N oder O und gegebenenfalls mit O, Cyclopropyl, Halogen oder Methyl substituiert, wenn Z C₍₂₋₆₎-Alkenyl ist, es gegebenenfalls mit Methyl substituiert ist; und
eines aus X und Y gleich CH ist und das andere N ist.

2. Eine Verbindung der Formel (I) gemäß Anspruch 1 oder ein Salz davon, wobei:
R¹ C₍₁₋₃₎-Alkoxy ist;
R² Cyano oder Chlor ist;
R³ gleich Z-COOH ist; und
Z C₍₂₋₃₎-Alkyl ist;
A (a) oder (b) ist;
B (d), (f) oder (g) ist; und
eines aus X und Y gleich CH ist und das andere N ist.

3. Eine Verbindung der Formel (I) gemäß Anspruch 1, ausgewählt aus:
3-[4-(5-{3-Chlor-4-[(1-methylethyl)oxy]phenyl}-1,2,4-oxadiazol-3-yl)-1*H*-pyrrolo[2,3-*b*]pyridin-1-yl]propansäure
3-[4-(5-{5-Chlor-6-[(1-methylethyl)oxy]-3-pyridinyl}-1,2,4-oxadiazol-3-yl)-1*H-*pyrrolo[2,3-*b*]pyridin-1-yl]propansäure
4-[4-(5-{3-Chlor-4-[(1-methylethyl)oxy]phenyl}-1,2,4-oxadiazol-3-yl)-1*H*-pyrrolo[2,3-*b*]pyridin-1-yl]butansäure
4-[4-(5-{5-Chlor-6-[(1-methylethyl)oxy]-3-pyridinyl}-1,2,4-oxadiazol-3-yl)-1*H-*pyrrolo[2,3-*b*]pyridin-1-yl]butansäure
3-[4-(5-{3-Chlor-4-[(1-methylethyl)oxy]phenyl}-1,2,4-oxadiazol-3-yl)-1*H*-pyrrolo[2,3-*c*]pyridin-1-yl]propansäure
4-[4-(5-{3-Chlor-4-[(1-methylethyl)oxy]phenyl}-1,2,4-oxadiazol-3-yl)-1*H-*pyrrolo[2,3-*c*]pyridin-1-yl]butansäure
3-[4-(5-{5-Chlor-6-[(1-methylethyl)oxy]-3-pyridinyl}-1,2,4-oxadiazol-3-yl)-1*H-*pyrrolo[2,3-*c*]pyridin-1-yl]propansäure
4-[4-(5-{5-Chlor-6-[(1-methylethyl)oxy]-3-pyridinyl}-1,2,4-oxadiazol-3-yl)-1*H-*pyrrolo[2,3-*c*]pyridin-1-yl]butansäure
3-[4-(5-{3-Chlor-4-[(1-methylethyl)oxy]phenyl}-1,3,4-thiadiazol-2-yl)-1*H*-pyrrolo[2,3-*b*]pyridin-1-yl]propansäure
4-[4-(5-{3-Chlor-4-[(1-methylethyl)oxy]phenyl}-1,3,4-thiadiazol-2-yl)-1*H*-pyrrolo[2,3-*b*]pyridin-1-yl]butansäure
3-[4-(5-{3-Chlor-4-[(1-methylethyl)oxy]phenyl}-1,3,4-oxadiazol-2-yl)-1*H*-pyrrolo[2,3-*b*]pyridin-1-yl]propansäure
4-[4-(5-{3-Chlor-4-[(1-methylethyl)oxy]phenyl}-1,3,4-oxadiazol-2-yl)-1*H*-pyrrolo[2,3-*b*]pyridin-1-yl]butansäure
3-[4-(2-{3-Chlor-4-[(1-methylethyl)oxy]phenyl}-1,3-thiazol-5-yl)-1*H*-pyrrolo[2,3-*b*]pyridin-1-yl]propansäure
4-[4-(2-{3-Chlor-4-[(1-methylethyl)oxy]phenyl}-1,3-thiazol-5-yl)-1*H*-pyrrolo[2,3-*b*]ppidin-1-yl]butansäure
und Salzen davon.

4. Verwendung einer Verbindung gemäß einem der Ansprüche 1 bis 3 zur Herstellung eines Medikaments zur Verwendung bei der Behandlung von Zuständen oder Störungen, die durch S1P1-Rezeptoren vermittelt werden.

5. Verwendung gemäß Anspruch 4, wobei der Zustand oder die Störung multiple Sklerose, Autoimmunerkrankungen, chronische Entzündungserkrankungen, Asthma, entzündliche Neuropathien, Arthritis, Transplantation, Morbus Crohn, Colitis ulcerosa, Lupus erythematodes, Psoriasis, Ischämie-Reperfusionsverletzung, solide Tumoren und Tumormetastase, Erkrankungen im Zusammenhang mit Angiogenese, Gefäßerkrankungen, Schmerzzustände, akute virale Erkrankungen, entzündliche Darmzustände, insulinabhängiger und nicht insulinabhängiger Diabetes ist.

6. Verwendung gemäß Anspruch 5, wobei der Zustand multiple Sklerose ist.

7. Ein Arzneimittel, umfassend eine Verbindung der Formel (I) oder ein pharmazeutisch verträgliches Salz davon gemäß einem der Ansprüche 1 bis 3.

## Revendications

1. Composé de formule (I) ou un de ses sels : 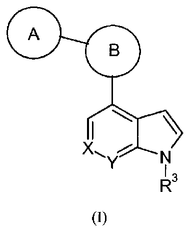 formule dans laquelle
A représente un noyau aromatique choisi entre : 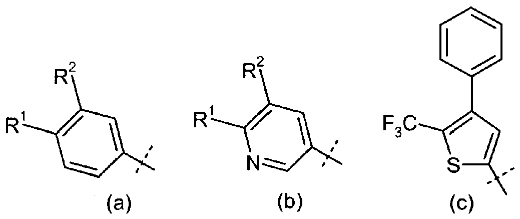
R¹ représente un groupe alkoxy en C₁ à C₆ ou alkyle en C₁ à C₆ ;
R² représente un groupe halogéno, cyano ou CF₃ ;
R³ représente un groupe Z-COOH ou (alkyle en C₁ à C₆) OH ;
B représente un noyau hétéroaryle pentagonal choisi entre :
* indique le point de fixation au noyau A ;
Z représente un groupe alkyle en C₁ à C₆ ou alcényle en C₂ à C₆ ;
lorsque Z représente un groupe alkyle en C₁ à C₆, il est facultativement interrompu par un groupe cyclopropyle, pipéridinyle, azétidinyle, pyrrolidinyle, facultativement interrompu par N ou O et facultativement substitué avec un substituant O, cyclopropyle, halogéno ou méthyle ; lorsque Z représente un groupe alcényle en C₂ à C₆, il est facultativement substitué avec un substituant méthyle ; et un de X et Y représente un groupe CH et l'autre représente N.

2. Composé de formule (I) suivant la revendication 1 ou un de ses sels, dans lequel :
R¹ représente un groupe alkoxy en C₁ à C₃ ;
R² représente un groupe cyano ou chloro ;
R³ représente un groupe Z-COOH ; et
Z représente un groupe alkyle en C₂ ou C₃ ;
A représente (a) ou (b) ;
B représente (d), (f) ou (g) ; et
un de X et Y représente un groupe CH et l'autre représente N.

3. Composé de formule (I) suivant la revendication 1, choisi entre :
l'acide 3-[4-(5-{3-chloro-4-[(1-méthyléthyl)oxy]phényl}-1,2,4-oxadiazol-3-yl)-1*H*-pyrrolo[2,3-*b*]pyridine-1-yl]propanoïque
l'acide 3-[4-(5-{5-chloro-6-[(1-méthyléthyl)oxy]-3-pyridinyl}-1,2,4-oxadiazol-3-yl)-1H-pyrrolo[2,3-b]pyridine-1-yl]propanoïque
l'acide 4-[4-(5-{3-chloro-4-[(1-méthyléthyl)oxy]phényl}-1,2,4-oxadiazol-3-yl)-1H-pyrrolo[2,3-b]pyridine-1-yl]butanoïque
l'acide 4-[4-(5-{5-chloro-6-[(1-méthyléthyl)oxy]-3-pyridinyl}-1,2,4-oxadiazol-3-yl)-1*H*-pyrrolo[2,3-b]pyridine-1-yl]butanoïque
l'acide 3-[4-(5-{3-chloro-4-[(1-méthyléthyl)oxy]phényl}-1,2,4-oxadiazol-3-yl)-1*H*-pyrrolo[2,3-*c*]pyridine-1-yl]propanoïque
l'acide 4-[4-(5-{3-chloro-4-[(1-méthyléthyl)oxy]phényl}-1,2,4-oxadiazol-3-yl)-1*H*-pyrrolo[2,3-*c*]pyridine-1-yl]butanoïque
l'acide 3-[4-(5-{5-chloro-6-[(1-méthyléthyl)oxy]-3-pyridinyl}-1,2,4-oxadiazol-3-yl)-1*H*-pyrrolo[2,3-*c*]pyridine-1-yl]propanoïque
l'acide 3-[4-(5-{3-chloro-4-[(1-méthyléthyl)oxy]phényl}-1,3,4-thiadiazol-2-yl)-1*H*-pyrrolo[2,3-*b*]pyridine-1-yl]propanoïque
l'acide 4-[4-(5-{3-chloro-4-[(1-méthyléthyl)oxy]phényl}-1,3,4-thiadiazol-2-yl)-1*H*-pyrrolo[2,3-*b*]pyridine-1-yl]butanoïque
l'acide 3-[4-(5-{3-chloro-4-[(1-méthyléthyl)oxy]phényl}-1,3,4-oxadiazol-2-yl)-1*H*-pyrrolo[2,3-*b*]pyridine-1-yl]propanoïque
l'acide 4-[4-(5-{3-chloro-4-[(1-méthyléthyl)oxy]phényl}-1,3,4-oxadiazol-2-yl)-1*H*-pyrrolo[2,3-*b*]pyridine-1-yl]butanoïque
l'acide 3-[4-(2-{3-chloro-4-[(1-méthyléthyl)oxy]phényl}-1,3-thiazol-5-yl)-1*H*-pyrrolo[2,3-*b*]pyridine-1-yl]propanoïque
l'acide 4-[4-(2-{3-chloro-4-[(1-méthyléthyl)oxy]phényl}-1,3-thiazol-5-yl)-1*H*-pyrrolo[2,3-*b*]pyridine-1-yl]butanoïque et ses sels.

4. Utilisation d'un composé suivant l'une quelconque des revendications 1 à 3 pour la production d'un médicament pour une utilisation dans le traitement d'affections ou de troubles à médiation par les récepteurs S1P1.

5. Utilisation suivant la revendication 4, dans laquelle l'affection ou le trouble est la sclérose en plaques, des maladies auto-immunes, des troubles inflammatoires chroniques, l'asthme, des neuropathies inflammatoires, l'arthrite, une transplantation, la maladie de Crohn, la colite ulcérative, le lupus érythémateux, le psoriasis, une lésion de reperfusion ischémique, des tumeurs solides, une métastase tumorale, des maladies associées à l'angiogénèse, des maladies vasculaires, des états douloureux, des maladies virales aigues, des affections intestinales inflammatoires, le diabète insulino-dépendant ou le diabète non insulino-dépendant.

6. Utilisation suivant la revendication 5, dans laquelle l'affection est la sclérose en plaques.

7. Composition pharmaceutique comprenant un composé de formule (I) ou un de ses sels pharmaceutiquement acceptables suivant l'une quelconque des revendications 1 à 3.
